# EUROPEAN PATENT APPLICATION

(11) **EP 4 299 712 A1**
(43) Date of publication of application: **03.01.2024**
(21) Application number: 21928142.5
(22) Date of filing: 28.12.2021
(51) Int. Cl.: C12M 1/34, C12M 1/38, C12M 3/00, C12N 5/078

(54) **CELL PROCESSING SYSTEM, CELL PROCESSING METHOD, AND LEARNING DATA CREATION METHOD**

(30) Priority: 24.02.2021 JP 2021027388
(71) Applicant: Sony Group Corporation, Tokyo 108-0075 (JP)
(72) Inventor: MATSUMOTO, Masahiro, Tokyo 108-0075 (JP); NAKAGAWA, Kazuhiro, Tokyo 108-0075 (JP)
(74) Representative: D Young & Co LLP
(86) International application number: PCT/JP2021/048796
(87) International publication number: WO 2022/181049

(57) **Abstract**

It is an object to provide an observation method for improving culturing efficiency of cells and obtaining desired cells without cost.

The present technology provides a cell treatment system including a cell culture unit capable of culturing a cell group, a measurement unit capable of measuring the cell group, and an estimation unit, in which the estimation unit estimates, on the basis of information regarding a cell before treatment of the cell group and at least one of information regarding a predetermined cell to reach after treatment of the cell group or a predetermined treatment condition for the cell group before treatment, a treatment condition under which the cell group is derivable to the information regarding the predetermined cell or information regarding the cell after treatment of the cell group derived by the predetermined treatment condition.

## Description

### TECHNICAL FIELD

The present invention relates to a cell treatment system, a cell treatment method, and a learning data creation method.

### BACKGROUND ART

In general, a technique for evaluating a culture state of a cell is used in an advanced medical field such as regenerative medicine and cell therapy.

For example, Patent Document 1 below discloses a sample holding unit capable of performing cell sorting, culture, cell processing, and the like in one space, and capable of changing the volume of the space to a size suitable for each step.

In addition, Patent Document 2 below discloses a method for analyzing and fractionating cells using a product obtained by forming a film of a cell adhesive light control material in which a cell adhesive material is bonded to a cell non-adhesive material via a photodissociable group. According to Patent Document 2 below, since the substrate can be irreversibly changed from cell adhesiveness to non-adhesiveness by a photodissociation reaction, the substrate is excellent in adhesion selectivity between cells and the substrate, and the purity, recovery rate, and the like of cells can be increased.

Furthermore, Patent Document 3 below discloses a culture assistance apparatus including an imaging unit that captures an image of a cell in culture at every predetermined time interval, a recording control unit that stores event information regarding culture such as medium replacement, passage, and cleaning inside the apparatus, and a learning unit that causes learning of a relevance between the captured image and the event information.

### CITATION LIST

### PATENT DOCUMENT

Patent Document 1: International Publication No. 2017/169259
Patent Document 2: International Publication No. 2011/058721
Patent Document 3: International Publication No. 2018/142702

### SUMMARY OF THE INVENTION

### PROBLEMS TO BE SOLVED BY THE INVENTION

For example, the observation method of the culture vessel described above requires observation over time after cell culture, and thus there is a case where cost is required in a case of processing complicated cell production. Therefore, there is a demand for an observation method for improving culturing efficiency of cells and obtaining desired cells without cost.

### SOLUTIONS TO PROBLEMS

The present inventors have found that culture efficiency is improved by estimating information regarding cells after treatment from culture conditions that has to reach predetermined cells or predetermined culture conditions from information regarding cells before treatment.

That is, the present technology provides a cell treatment system including:
a cell culture unit capable of culturing a cell group;
a measurement unit capable of measuring the cell group; and
an estimation unit, in which
the estimation unit estimates, on the basis of information regarding a cell before treatment of the cell group and at least one of information regarding a predetermined cell to reach after treatment of the cell group or a predetermined treatment condition for the cell group before treatment, a treatment condition under which the cell group is derivable to the information regarding the predetermined cell or information regarding the cell after treatment of the cell group derived by the predetermined treatment condition.

The estimation unit may set the information regarding the predetermined cell and/or the predetermined treatment condition on the basis of information regarding approval of the cell that is commercialized.

The measurement unit may be configured to acquire at least one of the information regarding the cell before treatment, the information regarding the cell after treatment, or information regarding the cell while the cell group is in treatment.

The measurement unit may include at least one of an image acquisition unit or a signal detection unit.

The image acquired by the image acquisition unit may be a stained image and/or a non-stained image.

The stained image may include a fluorescence image.

The non-stained image may include at least one of a bright field image, a phase difference image, a polarized image, or an image that is identified from information learned from the non-stained image or the fluorescence image and is pseudo-stained for each cell feature.

The image acquisition unit may acquire an image from at least one of a CMOS, a signal processing sensor, or an event detection sensor.

There may be further included a control unit that performs control to sort the cells on the basis of the predetermined treatment condition or the treatment condition estimated by the estimation unit.

There may be further included a control unit that perform control to culture the cell on the basis of the predetermined treatment condition or the treatment condition estimated by the estimation unit.

There may be further included a control unit that perform control to sort and culture the cell on the basis of the predetermined treatment condition or the treatment condition estimated by the estimation unit.

The control unit may set a threshold value for the estimated treatment condition or the information regarding the treated cell, and perform processing of stopping the production of the cell and/or re-collecting the cell depending on the threshold value or depending on information specified by a user.

The control unit may treat the cells fixed to the culture vessel via a light-selective linker in such a manner that the cells are sorted by optical control.

The culture control may control at least one of a physicochemical environment or a physiological environment.

The control of the physicochemical environment may include control by at least one of humidity, pH, osmotic pressure, oxygen partial pressure, or carbon dioxide partial pressure.

The control of the physiological environment may include control by at least one of culture solution, a stimulating factor, a transcription factor, or cell density.

The cell treatment system may further include a first learner that learns relevance between the cell treated under the treatment condition estimated by the estimation unit and information regarding therapy using the cell as input information.

The estimation unit may estimate information regarding therapy of the cell after treatment of the cell group derived by the treatment condition on the basis of the first learner and the information regarding the cell before treatment.

The cell treatment system may further include a second learner that learns relevance between the information regarding the cell before treatment and the treatment condition by using, as input information, the information regarding the cell before treatment, the treatment condition estimated by the estimation unit, and the cell before treatment treated under the treatment condition.

The estimation unit may estimate a treatment condition under which the cell group is derivable to the information regarding the predetermined cell on the basis of the second learner and the information regarding the cell before treatment.

The information regarding the cell before treatment may include at least one of a configuration, a state, a number, a proportion, a type, an increase rate, a survival rate, genetic information, or information regarding a molecule of the cell before treatment.

The information regarding the molecule may include at least one of a gene expression control factor such as a transcription factor or a transcription control factor, information regarding a molecular marker, or information regarding a cell surface antigen.

The information regarding the cell after treatment may include at least one of a configuration, a state, a number, a proportion, an increase rate, a survival rate, a response rate, a recurrence rate, or a side effect of the cell after treatment.

The treatment condition may include a culture condition related to at least one of a stimulating factor or a number of culture days.

The cell group may include a peripheral blood mononuclear cell fraction collected from a patient or a donor.

Furthermore, the present technology also provides an information processing device including:
an estimation unit that estimates, on the basis of
information regarding a cell before treatment of a cell group, and at least one of information regarding a predetermined cell to reach after treatment of the cell group or a predetermined treatment condition for the cell group before treatment,
a treatment condition under which the cell group is derivable to the information regarding the predetermined cell, or information regarding the cells after treatment of the cell group derived by the predetermined treatment condition.

Furthermore, the present technology also provides a learning data creation method including learning, by using a cell treated under a treatment condition estimated by an information processing device and information regarding a therapy using the cell as input information, relevance between the cell and the information regarding the therapy.

Furthermore, the present technology also provides a learning data creation method including learning, by using information regarding a cell before treatment, a treatment condition estimated by an information processing device, and the cell before treatment treated under the treatment condition as input information, relevance between the information regarding the cell before treatment and the treatment condition.

Furthermore, the present technology also provides a cell treatment method, including:
an estimation step of estimating, on the basis of information regarding a cell before treatment of a cell group included in a sample and at least one of information regarding a predetermined cell to reach after treatment of the cell group set in advance or a predetermined treatment condition for the cell group before treatment,
a treatment condition under which the cell group is derivable to information regarding the predetermined cell or information regarding the cell after treatment of the cell group derived under the predetermined treatment condition.

### BRIEF DESCRIPTION OF DRAWINGS

Fig. 1 is a schematic diagram of an example of a cell treatment system according to the present technology.
Fig. 2 illustrates an example of a block diagram of each of a cell culture unit, an information processing unit, and a measurement unit.
Fig. 3 is a schematic diagram of a cell treatment vessel according to the present technology.
Fig. 4 is a schematic diagram of an immobilized first molecule according to the present technology.
Fig. 5 is a schematic diagram illustrating an example of a configuration of a cell treatment vessel according to the present technology.
Fig. 6 is a schematic diagram illustrating an example of a configuration of an optical control unit according to the present technology.
Fig. 7 is a diagram schematically illustrating an overall configuration of a microscope system.
Fig. 8 is a diagram schematically illustrating an overall configuration of a biological sample analyzer.
Fig. 9 is a flowchart of a cell treatment method according to the present technology.
Fig. 10 is a flowchart of a sample preparation step according to the present technology.
Fig. 11 is a flowchart of a step of acquiring information regarding a cell before treatment according to the present technology.
Fig. 12 is a flowchart of an estimation step according to the present technology.
Fig. 13 is a flowchart of a treatment step according to the present technology.
Fig. 14 is a schematic diagram for describing a configuration example of the cell treatment system of the present technology.
Fig. 15 is a schematic diagram for describing a configuration example of the cell treatment system of the present technology.
Fig. 16 is a schematic diagram for describing a configuration example of the cell treatment system of the present technology.
Fig. 17 is a diagram illustrating amounts of additives added to a medium of each well.
Fig. 18 is a diagram illustrating a fluorochrome-labeled antibody used in measurement by a flow cytometer.
Fig. 19 is a diagram for describing an outline of an experiment.
Fig. 20 is a graph illustrating an example of a change in measured cell composition.
Fig. 21A illustrates a part of a data set used for creating a learned model.
Fig. 21B illustrates a part of the data set used for creating a learned model.
Fig. 22A is a diagram for describing prediction accuracy of a generated learned model.
Fig. 22B is a diagram illustrating an example of contribution degree data regarding explanatory variables.
Fig. 23A is a diagram for describing prediction accuracy of the generated learned model.
Fig. 23B is a diagram illustrating an example of contribution degree data regarding explanatory variables.

### MODE FOR CARRYING OUT THE INVENTION

Hereinafter, preferred modes for implementing the present technology will be described. Note that embodiments hereinafter described are representative embodiments of the present technology, and the scope of the present technology is not limited only to them. Note that the present technology will be described in the following order.
1. First Embodiment (Cell Treatment System)
   (1) Details of Problems of Invention
   (2) Description of First Embodiment
   (3) Example of First Embodiment
      (3-1) Cell Culture Unit
      (3-2) Information Processing Unit
      (3-3) Measurement unit
2. Second Embodiment (Cell Treatment Method)
   (1-1) Sample Preparation Step
   (1-2) Step of Acquiring Information Regarding Cell Before Treatment
   (1-3) Estimation Step
   (1-4) Treatment Step
   (1-5) Step of Acquiring Information Regarding Cells After Treatment
   (1-6) Learning Step
3. Configuration Example of Cell Treatment System
4. Example

### 1. First Embodiment (Cell Treatment System)

### (1) Details of Problems of Invention

As described above, in general, a technique for evaluating a culture state of a cell is used in advanced medical fields such as regenerative medicine and cell therapy. For example, a method has been proposed in which cells are selected, specific cells are cultured and observed, and the cells are easily recovered without damaging the cultured cells.

For example, Patent Document 1 above discloses a sample holding unit capable of performing cell sorting, culture, cell treatment, and the like in one space, and capable of changing the volume of the space to a size suitable for each step.

In addition, Patent Document 2 above discloses a method for analyzing and fractionating cells using a product obtained by forming a film of a cell adhesive light control material in which a cell adhesive material is bonded to a cell non-adhesive material via a photodissociable group.

However, in Patent Documents 1 and 2 described above, a method capable of performing cell sorting, culture, recovery, and cell treatment by photocontrolling a cell-adhesive/non-adhesive base material has been proposed, but a method relating to a cell observation method has not been proposed.

Incidentally, in the cell therapy field, the initial cell configuration of a cell group before culture treatment is not uniform and varies depending on the patient, the collection date, the treatment history, and the like, and thus even when the culture treatment is performed under the same conditions, the final cell group configuration, state, number, and the like vary. For example, peripheral blood mononuclear cells (PBMCs) collected from a patient or a donor can be cultured and expanded and/or transfected to produce cells that attack specific cancer cells. However, the cell configuration and/or condition of PBMCs collected from a patient or donor varies depending on the patient or donor, and even of the same patient or donor, it varies depending on the condition of the patient or donor at the time of collection, and thus is heterogeneous. In the current cell therapy, even different cell groups before the culture treatment are produced under the same conditions, so that the final cell configuration and/or state after the completed culture treatment can be administered to the patient in a heterogeneous state. From this, it is considered that there are patients who cannot obtain a therapeutic effect due to heterogeneity of the cell group and patients who have large side effects.

As described above, in order to improve the response rate of cancer treatment or the like by using a cell group, particularly an immune cell group, as a therapeutic agent, it is necessary to homogenize components of the therapeutic agent, such as a cell configuration and a cell state, within a certain range. It is possible to take out a cell group in culture and adjust components by physical cell sorting using magnetic beads or a cell sorter. However, the component adjustment may cause a change in the cell state, a decrease in cell survival rate, and a loss in the number of cells, and may further complicate and increase the cost of complex and high-cost cell production.

As an approach to the above problem, for example, Patent Document 3 discloses a culture observation apparatus including an imaging unit that captures an image of cells in culture at every predetermined time interval, a recording control unit that stores event (medium exchange, passaging, in-device cleaning) information regarding culture, and a learning unit that learns a relevance between the captured image and the event information.

However, Patent Document 3 only discloses an observation method under a culture environment of a single cell type, and does not disclose a method for observing the cell configuration, state, and number of cell groups as described above.

Based on the above, an object of the present technology is to perform processing including culture control and cell sorting according to the cell configuration, state, and number of the cell group before the treatment, and to uniformize the cell configuration/state/number of the cell group after treatment.

### (2) Description of First Embodiment

A cell treatment system according to the present technology includes an estimation unit that estimates, on the basis of information regarding a cell before treatment of the cell group included in a sample and at least one of information regarding a predetermined cell to reach after treatment of the cell group set in advance or a predetermined treatment condition for the cell group before treatment, a treatment condition under which the cell group is derivable to information regarding the predetermined cell or information regarding the cell after treatment of the cell group derived under the predetermined treatment condition. According to the present system, it is possible to estimate information regarding a desired cell group and treatment conditions for achieving the desired cell group from information regarding cells before treatment in the cell group and information regarding predetermined cells and treatment conditions set by a user or the like, and it is possible to equalize the cell configuration/state/number of the final cell group after treatment efficiently and without cost without executing treatment such as a specific culture step.

In one embodiment of the present technology, the estimation unit can estimate a treatment condition under which the cell group is derivable to information regarding the predetermined cell. In particular, the estimation may be performed on the basis of information regarding cells before treatment of a cell group included in the sample and information regarding predetermined cells to reach after treatment of the cell group set in advance.

In another embodiment of the present technology, the estimation unit can estimate information regarding the treated cell of the cell group derived under the predetermined treatment condition. In particular, the estimation may be executed on the basis of information regarding cells of a cell group included in the sample before treatment and a predetermined treatment condition for the cell group before treatment.

In a preferred embodiment of the present technology, the cell treatment system may include a control unit that perform control to sort and/or culture the cell on the basis of a predetermined treatment condition or the treatment condition estimated by the estimation unit. Thus, it is possible to process the cell group, and by processing the cells under the treatment conditions estimated by the estimation unit or the treatment conditions designated by the user or the like, the cell configuration/state/number of the cell group after the final processing can be made uniform.

In a preferred embodiment of the present technology, the cell treatment method may further include a measurement step of acquiring information regarding the cell in treatment over time. This makes it possible to acquire information regarding the cell group over time in each treatment step, to observe the treatment situation of the cell group over time, and to improve the efficiency of the treatment step.

### (3) Example of First Embodiment

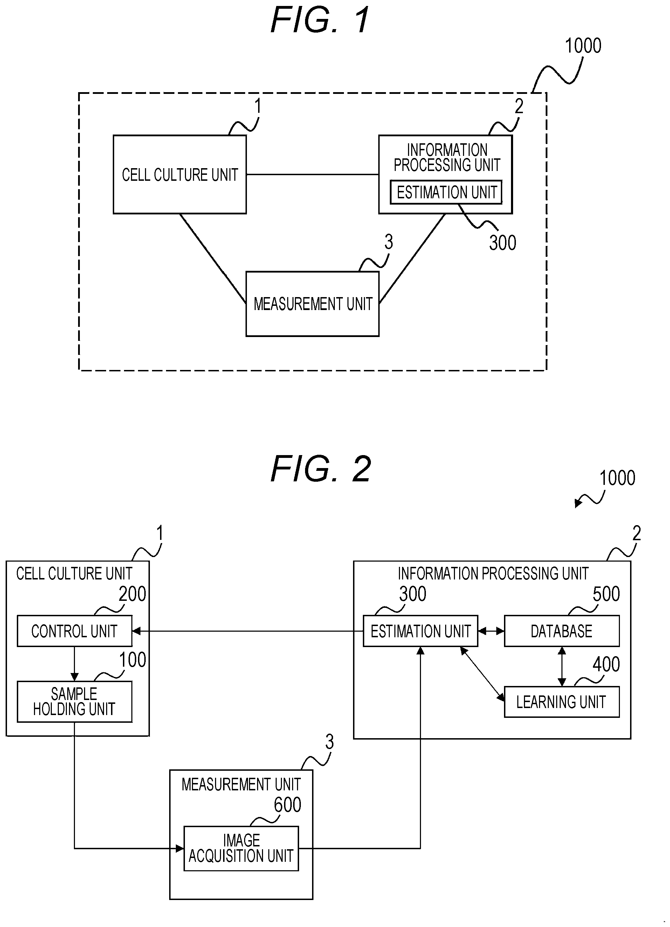

An example of a cell treatment system of the present technology is illustrated in Fig. 1. As illustrated in Fig. 1, the cell treatment system 1000 includes a cell culture unit 1, an information processing unit 2, and a measurement unit 3. The information processing unit 2 may include an estimation unit 300. The estimation unit 300 executes the estimation processing described in (2) above.

A configuration example of the cell treatment system 1000 is illustrated in Fig. 2. As illustrated in Fig. 2, the cell culture unit 1 may include, for example, a sample holding unit 100 and a control unit 200. As illustrated in the drawing, the information processing unit 2 may include, for example, an estimation unit 300 and a learning unit 400. The information processing unit 2 may further include a database 500. As illustrated in the drawing, the measurement unit 3 may include, for example, an image acquisition unit 600.

These will be described below.

### (3-1) Cell Culture Unit

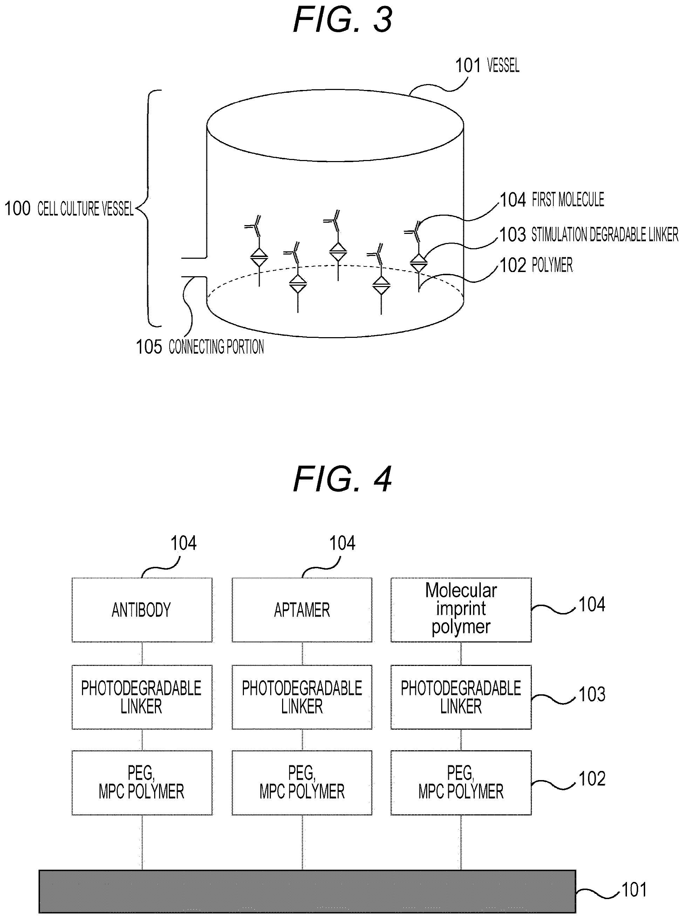

Fig. 3 illustrates a schematic diagram of the sample holding unit 100 of the cell culture unit 1. A first molecule capable of binding to the target cell is immobilized on the sample holding unit 100 via a degradable linker. The degradable linker may be fixed to a bottom surface of a vessel 101, as illustrated in Fig. 3. The sample holding unit 100 may be variable so that its volume increases or decreases. When the target cell is introduced into the culture vessel 101, the sample holding unit 100 captures the target cell with a first molecule 104 immobilized therein. The captured target cells are cultured in the culture vessel 101. In the present description, the target cell may mean a cell to be cultured among the cell group included in the sample.

The first molecule 104 is immobilized inside the culture vessel 101 of the sample holding unit 100 via, for example, a polymer 102 and a degradable linker 103. The degradable linker 103 may be directly immobilized without interposing the polymer 102. The immobilization is not limited to the bottom surface of the culture vessel 101, and may be performed on the inner wall, or in a case where a planar or three-dimensional internal structure is present inside the culture vessel 101, the immobilization may be performed on the surface of the structure. The inner surface of the culture vessel 101 is desirably coated with a substance suitable for cell survival (for example, collagen, fibroblast, and the like).

In a case where the polymer 102 is used, the polymer is preferably one that does not stress cells or is nontoxic or has biocompatibility. Examples of polymers include, for example, polyethylene glycol (PEG), 2-methacryloyloxyethyl phosphorylcholine polymers (MPC polymers).

In a case where the polymer 102 is used, the degradable linker 103 may be bonded to the end opposite to the position where the polymer is bonded to the culture vessel 101. Degradable linkers are molecules that degrade upon specific external stimuli. The degradable linker connects the first molecule 104 to the vessel bottom surface via the polymer 102 or in the diagram. Examples of the degradable linker include a linker that is degraded by light having a specific wavelength, a linker that is degraded by an enzyme, and a linker that is degraded by temperature, and the like. The degradable linker is preferably a photodegradable linker from the viewpoint that it can be controlled for each single cell (single cell) and the decomposition time is short.

The photodegradable linker is a molecule having a structure that is decomposed by light having a specific wavelength. The photodegradable linker may include, for example, as a group providing photodegradability, any of the following groups: a methoxynotrobenzyl group, a nitrobenzyl group (JP 2010-260831 A), a parahydroxyphenacyl group (Tetrahedron Letters, 1962, Vol. 1, p. 1), a 7-nitroindoline group (Journal of American Chemical Society, 1976, vol. 98, p. 843), a 2-(2-nitrophenyl) ethyl group (Tetrahedron, 1997, vol. 53, p. 4247), a (coumarin-4-yl) methyl group (Journal of American Chemical Society, 1984, vol. 106, p. 6860), and the like. Furthermore, the structural formulas of the photodegradable linkers that can be used in the present technology are illustrated in Table 1 below.

**[Table 1]**

| | | | |
|---|---|---|---|
| 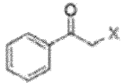 Section 2.1 | 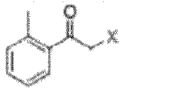 Section 2.2 | 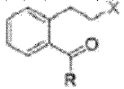 Section 2.2 | 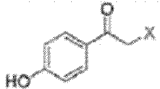 Section 2.3 |
| 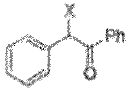 Section 2.4 | 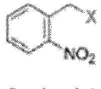 Section 3.1 | 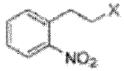 Section 3.2 | 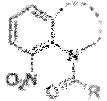 Section 3.3 |
| 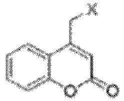 Section 4 | 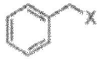 Section 5.1 | 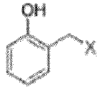 Section 5.2 | 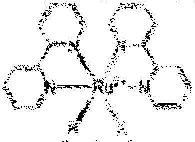 Section 6 |
| 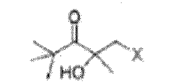 Section 7.1 | 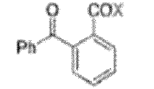 Section 7.2 | 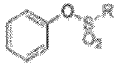 Section 73 | 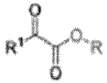 Section 1.4 |
| 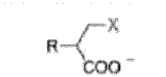 Section 7.5 | 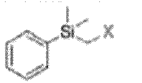 Section 7.6 | 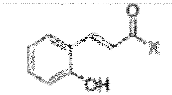 Section 7 7 | 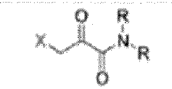 Section 7.8 |
| 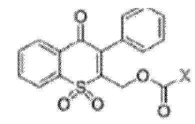 Section 7.9 | 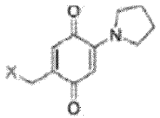 Section 7.10 | 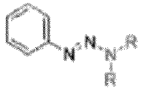 Section 7.11 | 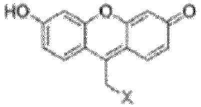 Section 7.12 |

| | | | |
|---|---|---|---|
| Photoremovable Protecting Groups in Chemistry and Biology: Reaction Mechanisms and Efficacy Chem. Rev. 2013, 113, 119-191 | | | |

The wavelength at which the photodegradable linker is decomposed approximately matches the absorption wavelength of the molecule. For example, in a case where the photodegradable linker contains a methoxynotrobenzyl group, if the absorption at 346 nm is 1, the photodegradable linker exhibits 0.89 absorption at 364 nm, 0.15 absorption at 406 nm, and 0.007 absorption at 487 nm. That is, when a light source having a wavelength of 365 nm is used, the photodegradable linker has good decomposition efficiency, and the photodegradable linker has a property of being hardly decomposed by a light source having a wavelength of 488 nm.

As described above, the wavelength of light applied to the photodegradable linker is only required to be a wavelength corresponding to each photodegradable linker. For example, the wavelength is around 330 to 450 nm. In addition, it is preferable to perform irradiation at, for example, 30 mW/cm^2, 100 sec. → 3 J/cm^2, which does not damage the cells. In particular, it is preferable not to use a wavelength of 300 nm or less because the wavelength may damage cells. With regard to the cytotoxicity by UV, it is said that DNA is damaged at 500 J/cm^2 and cell growth is inhibited depending on the type of cells (Callegari, A. J. & Kelly, T. J. Shedding light on the DNA damage checkpoint. Cell Cycle 6, 660-6 (2007)). Furthermore, there is also a report that cytotoxicity does not occur at 42 J/cm^2 (Masato T, et al, Optical cell separation from three-dimensional environment in photodegradable hydrogels for pure culture techniques, Scientific Reports 4, Article number. 4793 (2014)).

The first molecule 104 according to the present technology has a site capable of binding to a cell. As the site capable of binding to a cell, for example, an oleyl group, a cholesteryl group, an antibody, an aptamer, a molecular recognition polymer, or the like can be used.

The oleyl group and the cholesteryl group are hydrophobic and adhere to a floating cell surface. For example, a spacer such as PEG may be imparted to the oleyl group, and an NHS group (N-hydroxysuccinimide group) may be included at the terminal of the spacer to form the first molecule.

The antibody binds to a cell surface molecular antigen present in a target cell. Examples of the antibody include an antibody against a cancer-specific antigen, an antibody against a major histocompatibility antigen, and an antibody against a sugar chain, and the like.

The aptamer is a nucleic acid molecule or peptide that specifically binds to a molecule of the target cell. Examples of the aptamer include a DNA aptamer, an RNA aptamer, a peptide aptamer, a modified aptamer in which a modification is introduced into a nucleic acid skeleton or a base to improve specificity, and the like.

The molecular recognition polymer captures a target cell surface molecule with high selectivity even in the presence of a compound having physicochemical characteristics similar to those of a cell surface molecule of a target cell. The molecular recognition polymer, also referred to as a molecular imprinted polymer, has a selectively synthesized compound recognition region.

Fig. 4 illustrates a schematic example of a state in which the first molecule 104 is immobilized in the culture vessel 101. The first molecule 104 is immobilized on the bottom surface of the culture vessel 101 via the polymer 102 and the degradable linker 103. In Fig. 4, the first molecule 104 is directly bonded to the degradable linker 103, but may be bonded to the degradable linker 103 via a polymer. By adopting the above configuration, when the first molecule 104 approaches or comes into contact with the cell introduced into the culture vessel 101, the first molecule 104 can capture the cell.

The first molecule 104 is preferably immobilized such that one target cell is bound per spot. By capturing one cell per spot, cells having a molecule capable of binding to the first molecule 104 (antibodies, sugar chains, or the like) can be sorted at a single cell level. In addition, such selection can be executed for all spots.

In addition, the first molecules 104 are preferably immobilized in an array in the culture vessel 101. As a method of spotting the first molecules 104 in an array, a micro contact printing method, a spot method, or a method of decomposing an unnecessary portion after arranging the first molecules on the entire surface using the characteristics of the photodegradable linker may be used. With respect to the portion where the cells are desired to be prevented from being captured, the PEG or the MPC polymer described above may be coated or bound to the portion of the culture vessel 101, whereby nonspecific adsorption of the cells can be suppressed. Then, it is preferable that the photodegradable polymer is irradiated with light to liberate unnecessary cells, and then the PEG or the MPC polymer remains so that the cells are not non-specifically adsorbed to the released spot.

Note that the first molecule to be spotted may be one type, and for example, a first molecule configured to specifically capture only one type of cell may be adopted. Alternatively, the first molecule having different specificities may be immobilized for each spot, or different cells may be captured for each spot. In addition, the inside of the culture vessel 101 may be divided into sections, and first molecules having different specificities may be immobilized for each of the sections, whereby different cells can be captured for each of the sections in the same vessel. Alternatively, a first molecule that captures all kinds of cells may be immobilized, and in this case, cells may be sorted by a labeled second molecule described later. Use of a plurality of types of labeled second molecules enables multicolor analysis.

The culture vessel 101 may have a variable volume. In the culture vessel 101, it is preferable that the deformable portion is formed by a flexible material, and it is preferable that the deformable portion can expand and contract vertically and/or horizontally. The change in volume can be performed by adjusting the amount of liquid, the amount of air, or the like introduced from a connecting portion 105 provided in the culture vessel 101. In addition, the flow rate of the liquid or air supplied into the culture vessel 101 may be controlled so as to change the volume of the liquid (for example, a medium, a buffer solution, a staining buffer, or the like) in the culture vessel 101 without changing the volume. Thus, a reaction or the like can be performed at an optimum concentration for the cells in the vessel.

In order to allow the first molecules 104 to efficiently capture the target cells after the sample containing the target cells is put into the culture vessel 101, it is preferable to reduce the volume of the culture vessel 101 or the volume of the medium. This is because the contact probability between the target cell and the first molecule 104 increases when the volume or the medium volume is small. In addition, when the cell density increases after the target cell captured by the first molecule 104 is cultured, it is preferable to increase the volume of the culture vessel 101 or the volume of the medium. Increasing the volume or medium volume increases the cell culture space. Further culture solution can be added by increasing the volume.

Note that the culture vessel 101 preferably has gas permeability. Thus, for example, in a case where supply of oxygen and/or discharge of carbon dioxide are required in cell culture, the inside of the vessel can be brought into an environment suitable for cell culture (optimum CO2 concentration, optimum temperature, or the like). In particular, the surface on which the cells grow is preferably formed by, for example, a porous membrane or an oxygen permeable membrane.

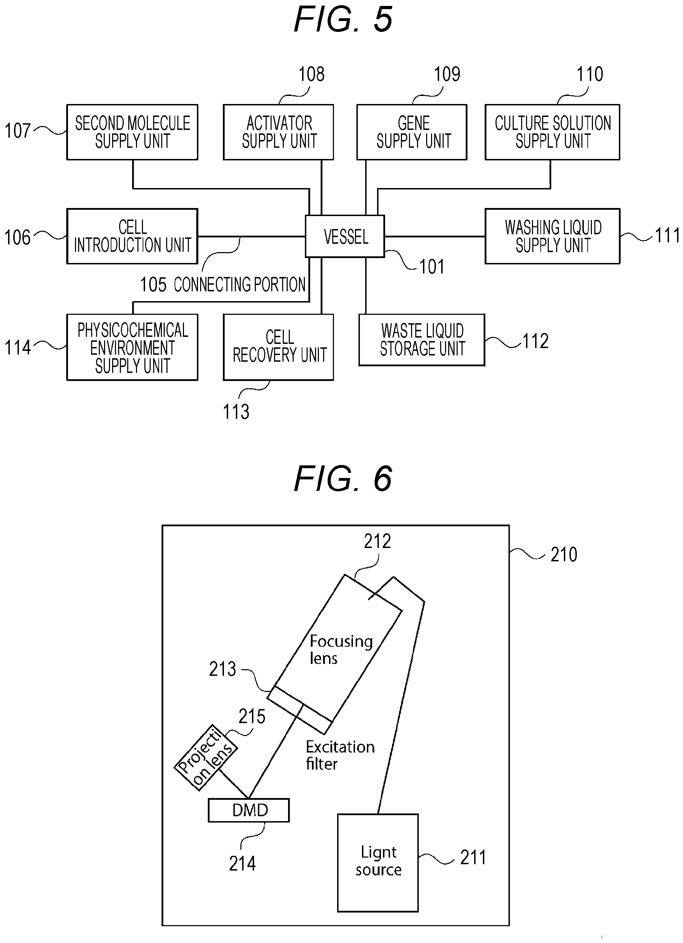

The culture vessel 101 may be connected to a vessel containing the sample containing the target cell or the culture solution described above by the connecting portion 105. For example, as illustrated in Fig. 5, any one or more of a cell introduction unit 106, a second molecule supply unit 107, an activator supply unit 108, a gene supply unit 109, a culture solution supply unit 110, a washing liquid supply unit 111, a waste liquid storage unit 112, and the cell recovery unit 113 may be connected to the culture vessel 101. All of these may be installed under conditions suitable for cell culture, or only the culture vessel 101 may be installed under conditions suitable for cell culture.

The cell introduction unit 106 holds a sample (particularly, a liquid sample) containing a target cell, and introduces the sample into the culture vessel 101. The type of the target cell is not particularly limited, and may be any of human-derived cells, immune cells, animal-derived cells, plant-derived cells, microorganism-derived cells, cancer cells, normal cells, stem cells, and epithelial cells. For example, in the case of animal cells, examples of the target cells include cells in blood and cells collected from biological tissues, and the like. Furthermore, the target cell may be either an adherent cell or a non-adherent cell. Furthermore, the number of types of target cells included in the sample may be one or more.

The second molecule supply unit 107 holds the second molecule therein, and is configured to introduce the second molecule into the culture vessel 101. In a case where a plurality of types of second molecules is required, the number of the second molecule supply units 107 may be increased according to the number of types of second molecules. The second molecule may be a molecule capable of binding to the target cell. The second molecule may be labeled with a fluorescent substance or the like. A structure in which the target cell is sandwiched between the first molecule and the second molecule (for example, a sandwich structure) is formed, and the formation of the structure can be recognized by the label. Similarly to the first molecule, the second molecule may be selected from the group including, for example, an oleyl group, an antibody, an aptamer, and a molecular recognition polymer. The second molecule may be a molecule that specifically binds to only a desired cell among the cells captured by the first molecule.

The label of the second molecule may be one fluorescent substance or a plurality of fluorescent substances. For example, one type of target cell may be recognized using one type of fluorescent substance. Alternatively, a plurality of types of target cells may be recognized using a plurality of types of fluorescent substances, and a method such as so-called polychromatic analysis may be used.

For a cell in which the second molecule cannot be recognized, a stimulus is applied to the degradable linker of the spot in which the cell is captured, and the linker is cleaved by the stimulus to release the cell. The released cells can be washed away from the culture vessel 101 with a washing liquid as an unnecessary substance. Without using the second molecule, a cell may be identified on the basis of an image that is identified from information learned from a bright field image, a phase difference image, a polarized image, and a non-stained image and a fluorescence image and is pseudo-stained for each feature of the cell, and the cell may be released by light stimulus. The identification may be performed by the information processing unit 2. The information processing unit 2 can drive the optical control unit on the basis of a result of the identification to apply the light stimulus. In the case of the present method, the second molecule is unnecessary, which can contribute to cost reduction of the process. In addition, since the staining step can be omitted, this contributes to shortening of the step.

The washing liquid supply unit 111 holds the washing liquid. A washing liquid is supplied when an unnecessary object or the like in the culture vessel 101 is cleaned. The washing liquid may be one generally used for cell culture and the like. The washing liquid is not particularly limited, and examples thereof include physiological saline, Tris buffer, HEPES buffer, purified water, and the like.

The activator supply unit 108 holds an activator that activates cells, particularly a liquid containing the activator. The activator supply unit 108 may be configured to supply an activator into the culture vessel 101. The activator may be selected according to the target cell, and is not particularly limited, and examples thereof include cytokines, hormones, interleukins, antibodies, and the like. The activator can activate cells before, during, or after culturing target cells.

The gene supply unit 109 holds a gene to be introduced into the target cell. The gene supply unit 109 may be configured to supply the gene into the culture vessel 101. The gene may be either an endogenous gene or a foreign gene. The gene may be incorporated into a phage vector, a plasmid vector, a viral vector, or the like suitable for gene introduction. For example, the gene supply unit 109 can supply a viral vector incorporating a target gene to the culture vessel 101. Thus, the target cell can be infected with the viral vector and subjected to gene introduction. Furthermore, the gene supply unit 109 may supply, for example, a genome editing reagent containing a specific base sequence and a specific enzyme, such as a CRISPR/Cas9 system, to the culture vessel 101, whereby the gene may be introduced into the target cell.

The culture solution supply unit 110 holds a culture solution suitable for the target cell and supplies the culture solution to the culture vessel 101. As the culture solution, one suitable for the target cell can be selected, and for example, Eagle's medium, D-MEM medium, E-MEM medium, RPMI-1640 medium, Dulbecco's PBS medium, or the like can be used. Note that when the culture solution is colored with phenol red or the like, the optimum pH range (for example, pH 6.8 to 7.2) of the culture solution can be managed during culture of the target cells in the culture vessel 101.

The waste liquid storage unit 112 temporarily receives waste liquid, culture solution, or the like containing the unnecessary substance. The waste liquid is subjected to sterilization treatment or the like as necessary, and then discarded.

The cell recovery unit 113 recovers and holds the cells cultured in the culture vessel 101. The recovery method is not particularly limited, but can be performed by suction or extrusion, or by disposing the cell recovery unit 113 below the culture vessel 101, or the like.

The physicochemical environment supply unit 114 holds or provides a physical environment of the sample holding unit 100. The physical environment during culture can be appropriately selected according to the target cell, and is not particularly limited, and examples thereof include humidity, pH, osmotic pressure, oxygen partial pressure, carbon dioxide partial pressure, and the like. Thus, an optimal physical environment can be provided for the target cell, and the survival rate of the cell can be increased.

The connecting portion 105 connects the culture vessel 101 and any one or more portions of the respective units 106 to 114 described above, and a liquid flows therethrough. For example, a tube is used as the connecting portion 105. As a liquid feeding method, a peristaltic pump that does not come into contact with liquid is preferable.

The control unit 200 of the cell culture unit 1 includes, for example, an optical control unit and an environment control unit. The control unit 200 performs physicochemical control on the cells and/or the environment around the cells included in the sample holding unit 100.

The optical control unit may apply a stimulus by irradiating the degradable linker with light having a specific wavelength corresponding to the degradable linker by optical control. The optical control unit may include, for example, a light source and a micro electro mechanical systems (MEMS) element for causing light emitted from the light source to reach a predetermined position (a predetermined position in the vessel 101). The MEMS element may be, for example, a DMD or a scanning mirror. The optical control unit may further include an optical element (for example, lenses, filters, mirrors, prisms, and the like) for controlling a shape and/or a wavelength of the light.

The environment control unit may hold or provide a physicochemical or physiological environment around cells included in the sample holding unit 100 by physicochemical or physiological control.

Fig. 6 illustrates an example of a configuration of the optical control unit. The optical control unit 210 illustrated in Fig. 6 includes a light source 211, a focusing lens 212, an excitation filter 213, a digital mirror device 214, and a projection lens 215 in order to emit light of the specific wavelength. By having the above configuration, unnecessary cells can be selected and released. The light source 211 emits light having a wavelength corresponding to the photodegradable linker. The focusing lens 212 focuses the light, and the excitation filter 213 extracts and transmits only light of a specific wavelength. The digital mirror device 214 includes a movable micromirror, and can selectively irradiate each spot on which the first molecule is immobilized with light by tilting each micromirror. The projection lens 215 emits light reflected by digital mirror device 214 toward a surface of the culture vessel 101 having a spot on which the first molecule is immobilized. By the selective light irradiation by the optical control unit 210, it is possible to selectively decompose the degradable linker of the spot in which the cell to be released is captured.

The optical control unit 210 can further include a stimulus control unit capable of stimulating the degradable linker for each of the first spots. In a case where the optical control unit 210 is a light irradiation device, for example, it is sufficient that the cells arranged in the culture vessel 101 can be individually irradiated with light at intervals on the order of several tens of um, and thus, for example, a digital micromirror device (DMD), a liquid crystal panel, a MEMS shutter, or the like can be used. The excitation filter 213 is disposed between the light source 211 and the DMD 214, and a mechanism such as rotation of the filter is added so as to obtain an optimum filter configuration according to a purpose, whereby multicolor analysis can be supported. Since a DMD including a full HD number of micromirrors is commercially available, a stimulus control unit of a light irradiation device using the DMD can simultaneously control 1920 × 1080 sites (on/off of irradiation). Therefore, individual control to about 2 × 10⁶ cells is simultaneously possible. For example, in a case where cells (Φ30 um or less) are aligned at a pitch of 30 um at 1920 × 1080, an area of about 58 × 33 mm is required. In a case where it is desired to process 10⁷ cells, 10 cells are prepared. In a case where five surfaces are arranged in two rows, the size is 116 × 165 mm, which is slightly smaller than the B6 size and is a size that can be realized for 2 × 10⁷ cell analysis.

The environment control unit controls a physicochemical environment and/or a physiological environment in the vessel 101.

In order to control the physicochemical environment, the environment control unit can control the physicochemical environment supply unit 114, thereby controlling humidity, pH, osmotic pressure, oxygen partial pressure, carbon dioxide partial pressure, or the like in the vessel 101. Thus, an optimum physical environment can be provided to the target cell in the culture environment, and the survival rate of the cell can be increased.

In order to control the physiological environment, the environment control unit can control at least one of the activator supply unit 108, the gene supply unit 109, or the culture solution supply unit 110. Thus, an optimum physiological environment is provided to the target cell in the culture environment, and the culture efficiency of the cell can be improved. The control of the physiological environment may include control by at least one of culture solution, a stimulating factor, a transcription factor, or cell density.

### (3-2) Information Processing Unit

The cell treatment system 1000 includes the information processing unit 2 including the estimation unit 300, the learning unit 400, and the database 500.

The estimation unit 300 estimates, on the basis of information regarding a cell before treatment of a cell group included in a sample and at least one of information regarding a predetermined cell to reach after treatment of the cell group set in advance or a predetermined treatment condition for the cell group before the treatment, a treatment condition under which the cell group is derivable to information regarding the predetermined cell or information regarding the cell after treatment of the cell group derived under the predetermined treatment condition. Thus, it is possible to estimate information regarding a desired cell group and treatment conditions for achieving the desired cell group from information regarding cells before treatment in the cell group and information regarding predetermined cells or treatment conditions set by a user or the like, and it is possible to equalize the cell configuration/state/number of the final cell group after treatment efficiently and without increasing cost without executing treatment such as a specific culture step.

The learning unit 400 includes a first learner that learns the relevance between the cell and the information regarding therapy using the information regarding the cell treated under the treatment condition estimated by the estimation unit 300 and the information regarding therapy using the cell as input information for learning, and a second learner that learns the relevance between the information regarding the cell before the treatment and the treatment condition using the information regarding the cell before the treatment, the treatment condition estimated by the estimation unit 300, and the cell before the treatment treated under the treatment condition as input information for learning. This makes it possible to estimate, from the information regarding the cell before treatment in the cell group, the treatment conditions for achieving a desired cell group and the information regarding the cell group after treatment for achieving a desired therapeutic effect of the cell, and to increase the efficiency of the cell culture process.

The database 500 acquires and holds the treatment conditions of cells acquired by the cell culture unit 1, the information regarding cells acquired by the measurement unit 3, and the information regarding therapy of the cells acquired from the external database. The database 500 may be configured to transmit the information described above to the estimation unit 300 and the learning unit 400. Thus, the estimation unit 300 and the learning unit 400 can refer to the information held in the database 500, and estimation efficiency and learning efficiency of the estimation unit 300 and the learning unit 400 are improved.

The information processing unit 2 may be designed as a general-purpose computer, and may be designed as an information processing unit that includes a CPU, a RAM, and a ROM, for example. The information processing unit may be included in a housing in which the cell culture unit 1 and the measurement unit are included, or may be located outside the housing. Further, the various processes or functions to be executed by the information processing unit may be realized by a server computer or a cloud connected via a network.

### (3-3) Measurement unit

The cell treatment system 1000 includes a measurement unit 3 that acquires at least one of information regarding a cell before treatment, information regarding a cell after treatment, or information regarding a cell in treatment. Thus, efficiency of the treatment step by the sample processing unit is enhanced. The measurement unit 3 includes the image acquisition unit 600 that acquires information regarding a cell in treatment by the control unit 200 by acquiring an image. The image acquired by the image acquisition unit may be a stained image and/or a non-stained image. The stained image may include a fluorescence image. In addition, the non-stained image may include at least one of a bright field image, a phase difference image, a polarized image, or an image that is identified from information learned from the non-stained image or the fluorescence image and is pseudo-stained for each cell feature. Note that the measurement unit 3 may include a signal detection unit that acquires information regarding a cell in treatment by the sample processing unit by acquiring a signal in addition to the image acquisition unit 600 or instead of the image acquisition unit 600. For example, the image acquisition unit 600 can be configured as a microscope system described below in (3-3-1). In addition, in a case where the measurement unit 3 includes the signal detection unit, the measurement unit 3 may be configured as a biological sample analyzer. The biological sample analyzer will be described below in (3-3-2).

### (3-3-1) Microscope System

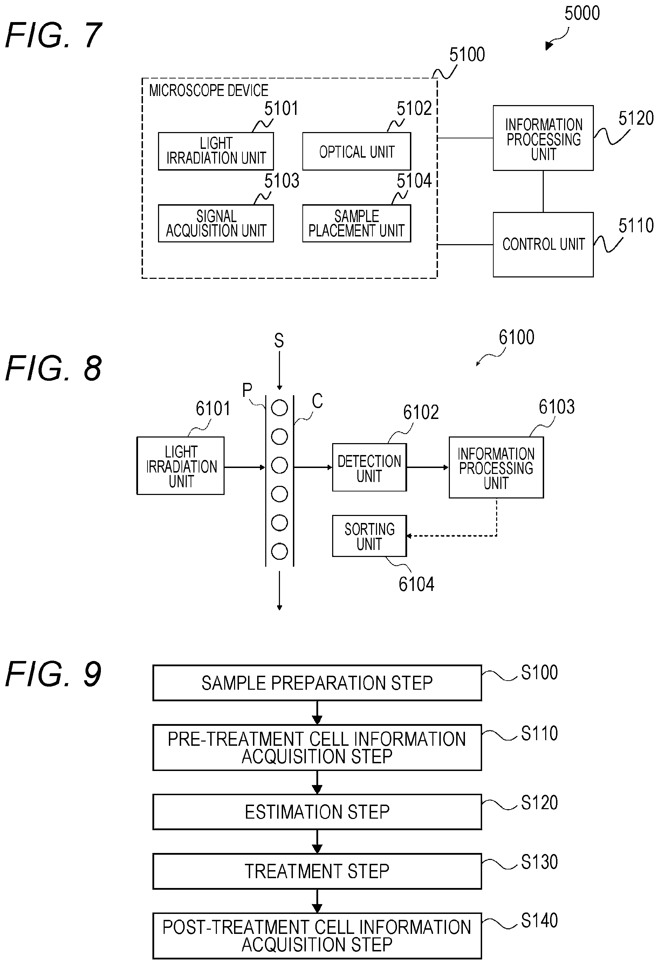

A configuration example of the microscope system is illustrated in Fig. 7. The microscope system 5000 illustrated in Fig. 7 includes a microscope device 5100, a control unit 5110, and an information processing unit 5120. The microscope device 5100 includes a light irradiation unit 5101, an optical unit 5102, and a signal acquisition unit 5103. The microscope device 5100 may further include a sample placement unit 5104 on which a biological sample S is placed. Note that the configuration of the microscope apparatus is not limited to that illustrated in Fig. 7, and for example, the light irradiation unit 5101 may exist outside the microscope device 5100, and for example, a light source not included in the microscope device 5100 may be used as the light irradiation unit 5101. Alternatively, the light irradiation unit 5101 may be disposed so that the sample placement unit 5104 is sandwiched between the light irradiation unit 5101 and the optical unit 5102, and may be disposed on the side at which the optical unit 5102 exists, for example. The microscope device 5100 may be configured by one or two or more of bright field observation, phase difference observation, differential interference observation, polarization observation, fluorescence observation, and dark field observation.

The microscope system 5000 may be configured as a so-called whole slide imaging (WSI) system or a digital personal computer system, and may be used for pathological diagnosis. Alternatively, the microscope system 5000 may be designed as a fluorescence imaging system, or particularly, as a multiple fluorescence imaging system.

For example, the microscope system 5000 may be used to make an intraoperative pathological diagnosis or a telepathological diagnosis. In the intraoperative pathological diagnosis, the microscope device 5100 can acquire the data of the biological sample S acquired from the subject of the operation while the operation is being performed, and then transmit the data to the information processing unit 5120. In the telepathological diagnosis, the microscope device 5100 can transmit the acquired data of the biological sample S to the information processing unit 5120 located in a place away from the microscope device 5100 (such as in another room or building). Then, in these diagnoses, the information processing unit 5120 receives and outputs the data. The user of the information processing unit 5120 can perform pathological diagnosis on the basis of the output data.

### (Biological Sample)

The biological sample S may be a sample containing a biological component. The biological component may be a tissue, a cell, a liquid component of the living body (blood, urine, or the like), a culture, or a living cell (a myocardial cell, a nerve cell, a fertilized egg, or the like).

The biological sample may be a solid, or may be a specimen fixed with a fixing reagent such as paraffin or a solid formed by freezing. The biological sample can be a section of the solid. A specific example of the biological sample may be a section of a biopsy sample.

The biological sample may be one that has been subjected to a treatment such as staining or labeling. The treatment may be staining for indicating the morphology of the biological component or for indicating the substance (surface antigen or the like) contained in the biological component, and can be hematoxylin-eosin (HE) staining or immunohistochemistry staining, for example. The biological sample may be one that has been subjected to the above treatment with one or more reagents, and the reagent(s) can be a fluorescent dye, a coloring reagent, a fluorescent protein, or a fluorescence-labeled antibody.

The specimen may be prepared from a specimen or a tissue sample collected from a human body for the purpose of pathological diagnosis or clinical examination. Alternatively, the specimen is not necessarily of the human body, and may be derived from an animal, a plant, or some other material. The specimen may differ in property, depending on the type of the tissue being used (such as an organ or a cell, for example), the type of the disease being examined, the attributes of the subject (such as age, gender, blood type, and race, for example), or the subject's daily habits (such as an eating habit, an exercise habit, and a smoking habit, for example). The specimen may be accompanied by identification information (bar code information, QR code (registered trademark) information, or the like) for identifying each specimen, and be managed in accordance with the identification information.

### (Light Irradiation Unit)

The light irradiation unit 5101 is a light source for illuminating the biological sample S, and is an optical unit that guides light emitted from the light source to a specimen. The light source can illuminate a biological sample with visible light, ultraviolet light, infrared light, or a combination thereof. The light source may be one or more of a halogen lamp, a laser light source, an LED lamp, a mercury lamp, and a xenon lamp. The light source in fluorescent observation may be of a plurality of types and/or wavelengths, and the types and the wavelengths may be appropriately selected by a person skilled in the art. The light irradiation unit may have a configuration of a transmissive type, a reflective type, or an epi-illumination type (a coaxial epi-illumination type or a side-illumination type).

### (Optical Unit)

The optical unit 5102 is designed to guide the light from the biological sample S to the signal acquisition unit 5103. The optical unit may be designed to enable the microscope device 5100 to observe or capture an image of the biological sample S.

The optical unit 5102 may include an objective lens. The type of the objective lens may be appropriately selected by a person skilled in the art, in accordance with the observation method. The optical unit may also include a relay lens for relaying an image magnified by the objective lens to the signal acquisition unit. The optical unit may further include optical components other than the objective lens and the relay lens, and the optical components may be an eyepiece, a phase plate, a condenser lens, and the like.

The optical unit 5102 may further include a wavelength separation unit designed to separate light having a predetermined wavelength from the light from the biological sample S. The wavelength separation unit may be designed to selectively cause light having a predetermined wavelength or a predetermined wavelength range to reach the signal acquisition unit. The wavelength separation unit may include one or more of the following: a filter, a polarizing plate, a prism (Wollaston prism), and a diffraction grating that selectively pass light, for example. The optical component(s) included in the wavelength separation unit may be disposed in the optical path from the objective lens to the signal acquisition unit, for example. The wavelength separation unit is provided in the microscope device in a case where fluorescent observation is performed, or particularly, where an excitation light irradiation unit is included. The wavelength separation unit may be designed to separate fluorescence or white light from fluorescence.

### (Signal Acquisition Unit)

The signal acquisition unit 5103 may be designed to receive light from the biological sample S, and convert the light into an electrical signal, or particularly, into a digital electrical signal. The signal acquisition unit may be designed to be capable of acquiring data about the biological sample S, on the basis of the electrical signal. The signal acquisition unit may be designed to be capable of acquiring data of an image (a captured image, or particularly, a still image, a time-lapse image, or a moving image) of the biological sample S, or particularly, may be designed to acquire data of an image enlarged by the optical unit. The signal acquisition unit includes one or more image sensors, CMOSs, CCDs, or the like that include a plurality of pixels arranged in one- or two-dimensional manner. The signal acquisition unit may include an image sensor for acquiring a low-resolution image and an image sensor for acquiring a high-resolution image, or may include an image sensor for sensing for AF or the like and an image sensor for outputting an image for observation or the like. The image sensor may be a signal processing sensor including not only the plurality of pixels, but also a signal processing unit (including one, two, or three of the following: a CPU, a DSP, and a memory) that performs signal processing using pixel signals from the respective pixels, and an output control unit that controls outputting of image data generated from the pixel signals and processed data generated by the signal processing unit. Furthermore, the imaging element may include an asynchronous event detection sensor that detects, as an event, that a luminance change of a pixel that photoelectrically converts incident light exceeds a predetermined threshold value. The image sensor including the plurality of pixels, the signal processing unit, and the output control unit can be preferably designed as a one-chip semiconductor device.

### (Control Unit)

The control unit 5110 controls imaging being performed by the microscope device 5100. For the imaging control, the control unit can drive movement of the optical unit 5102 and/or the sample placement unit 5104, to adjust the positional relationship between the optical unit and the sample placement unit. The control unit 5110 can move the optical unit and/or the sample placement unit in a direction toward or away from each other (in the optical axis direction of the objective lens, for example). The control unit may also move the optical unit and/or the sample placement unit in any direction in a plane perpendicular to the optical axis direction. For the imaging control, the control unit may control the light irradiation unit 5101 and/or the signal acquisition unit 5103.

### (Sample Placement Unit)

The sample placement unit 5104 may be designed to be capable of securing the position of a biological sample on the sample placement unit, and may be a so-called stage. The sample placement unit 5104 may be designed to be capable of moving the position of the biological sample in the optical axis direction of the objective lens and/or in a direction perpendicular to the optical axis direction.

### (Information Processing Unit)

The information processing unit 5120 can acquire, from the microscope device 5100, data (imaging data or the like) acquired by the microscope device 5100. The information processing unit can perform image processing on the imaging data. The image processing may include color separation processing. The color separation process may include a process of extracting data of the optical component of a predetermined wavelength or in a predetermined wavelength range from the imaging data to generate image data, or a process of removing data of the optical component of a predetermined wavelength or in a predetermined wavelength range from the imaging data. The image processing may also include an autofluorescence separation process for separating the autofluorescence component and the dye component of a tissue section, and a fluorescence separation process for separating wavelengths between dyes having different fluorescence wavelengths from each other. The autofluorescence separation process may include a process of removing the autofluorescence component from image information about another specimen, using an autofluorescence signal extracted from one specimen of the plurality of specimens having the same or similar properties.

The information processing unit 5120 may transmit data for the imaging control to the control unit 5110, and the control unit 5110 that has received the data may control the imaging being by the microscope device 5100 in accordance with the data.

The information processing unit 5120 may be designed as an information processing unit such as a general-purpose computer, and may include a CPU, RAM, and ROM. The information processing unit may be included in the housing of the microscope device 5100, or may be located outside the housing. Further, the various processes or functions to be executed by the information processing unit may be realized by a server computer or a cloud connected via a network.

Note that the image acquired by the signal acquisition unit 5103 may be a stained image and/or a non-stained image. The signal acquisition unit may acquire information regarding the cells before, during, and after treatment as the feature quantity from the image. Specific examples of the information regarding the cell are as described later.

The stained image is, for example, a fluorescence image obtained by irradiating the biological sample S stained with a fluorescent reagent with excitation light by the light irradiation unit 5101. This makes molecular marker analysis of the biological sample S having a biomarker such as CD4 or CD8 simple and quantitative. The non-stained image may be a bright field image, a phase difference image, or a polarized image obtained from the non-stained biological sample S. Furthermore, the non-stained image may be an image that is identified from information learned from the non-stained image and the fluorescence image and is pseudo-stained for each cell feature. In the pseudo-stained image, it is possible to predict various labels such as nuclei, cell types (nerves and the like), and cell states (cell death and the like) from the non-stained image, and it is possible to eliminate the limitation of the number of simultaneous labels due to overlapping of fluorescence spectra due to chemical staining. A specific method of the pseudo-stained image is not particularly limited as long as it is a known method, and examples thereof include the following methods (Cell. 2018 Apr 19; 173(3): 792 to 803. e19. doi: 10.1016/j.cell. 2018.03.040. Epub 2018 Apr 12).

### (3-3-2) Biological sample analyzer

A configuration example of the biological sample analyzer is illustrated in Fig. 8. A biological sample analyzer 6100 shown in Fig. 8 includes: a light irradiation unit 6101 that irradiates a biological sample S flowing in a flow channel C with light; a detection unit 6102 that detects light generated by the irradiation, and an information processing unit 6103 that processes information about the light detected by the detection unit. The biological sample analyzer 6100 is a flow cytometer or an imaging cytometer, for example. The biological sample analyzer 6100 may include a sorting unit 6104 that sorts out specific biological particles P in a biological sample. The biological sample analyzer 6100 including the sorting unit is a cell sorter, for example.

### (Biological Sample)

The biological sample S may be a liquid sample containing biological particles. The biological particles are cells or non-cellular biological particles, for example. The cells may be living cells, and more specific examples thereof include blood cells such as erythrocytes and leukocytes, and germ cells such as sperms and fertilized eggs. Also, the cells may be those directly collected from a sample such as whole blood, or may be cultured cells obtained after culturing. The non-cellular biological particles are extracellular vesicles, or particularly, exosomes and microvesicles, for example. The biological particles may be labeled with one or more labeling substances (such as a dye (particularly, a fluorescent dye) and a fluorochrome-labeled antibody). Note that particles other than biological particles may be analyzed by the biological sample analyzer of the present disclosure, and beads or the like may be analyzed for calibration or the like.

### (Flow Channel)

The flow channel C may be designed so that a biological sample flows, and in particular, a flow in which the biological particles contained in the biological sample are aligned substantially in one row is formed. The flow channel structure including the flow channel C may be designed so that a laminar flow is formed, and in particular, is designed so that a laminar flow in which the flow of the biological sample (a sample flow) is surrounded by the flow of a sheath liquid is formed. The design of the flow channel structure may be appropriately selected by a person skilled in the art, or a known one may be adopted. The flow channel C may be formed in a flow channel structure such as a microchip (a chip having a flow channel on the order of micrometers) or a flow cell. The width of the flow channel C is 1 mm or smaller, or particularly, may be not smaller than 10 um and not greater than 1 mm. The flow channel C and the flow channel structure including the flow channel C may be made of a material such as plastic or glass.

The device of the present disclosure may be designed so that the biological sample flowing in the flow channel C, or particularly, the biological particles in the biological sample are irradiated with light from the light irradiation unit. The device of the present disclosure may be designed so that the irradiation point of light on the biological sample is located in the flow channel structure in which the flow channel C is formed, or may be designed so that the irradiation point is located outside the flow channel structure. An example of the former case may be a configuration in which the light is emitted onto the flow channel C in a microchip or a flow cell. In the latter case, the biological particles after exiting the flow channel structure (particularly, the nozzle portion thereof) may be irradiated with the light, and a flow cytometer of a jet-in-air type can be adopted, for example.

### (Light Irradiation Unit)

The light irradiation unit 6101 includes a light source unit that emits light, and a light guide optical system that guides the light to the flow channel C. The light source unit includes one or more light sources. The type of the light source(s) may be a laser light source or an LED, for example. The wavelength of light to be emitted from each light source may be any wavelength of ultraviolet light, visible light, and infrared light. The light guide optical system includes optical components such as beam splitters, mirrors, or optical fibers, for example. The light guide optical system may also include a lens group for condensing light, and may include an objective lens, for example. The biological sample may be irradiated with light at one or more irradiation points. The light irradiation unit 5101 may be designed to collect light emitted onto one irradiation point from one light source or different light sources.

### (Detection Unit)

The detection unit 6102 includes at least one photodetector that detects light generated by emitting light onto particles by the light irradiation unit. The light to be detected may be fluorescence or scattered light (such as one or more of the following: forward scattered light, backscattered light, and side scattered light), for example. Each photodetector includes one or more light receiving elements, and has a light receiving element array, for example. Each photodetector may include one or more photomultiplier tubes (PMTs) and/or photodiodes such as APDs and MPPCs, as the light receiving elements. The photodetector includes a PMT array in which a plurality of PMTs is arranged in a one-dimensional direction, for example. The detection unit may also include an image sensor such as a CCD or a CMOS. With the image sensor, the detection unit can acquire an image (such as a bright field image, a dark-field image, or a fluorescent image, for example) of biological particles.

The detection unit includes a detection optical system that causes light of a predetermined detection wavelength to reach the corresponding photodetector. The detection optical system includes a spectroscopic unit such as a prism or a diffraction grating, or a wavelength separation unit such as a dichroic mirror or an optical filter. For example, the detection optical system may be configured to disperse light from the biological particle, and detect light in different wavelength regions by a plurality of photodetectors having a number larger than the number of fluorescent dyes. A flow cytometer including such a detection optical system is called a spectral flow cytometer. Further, the detection optical system may be designed to separate the light corresponding to the fluorescence wavelength band of a specific fluorescent dye from the light from the biological particles, for example, and cause the corresponding photodetector to detect the separated light.

The detection unit may also include a signal processing unit that converts an electrical signal obtained by a photodetector into a digital signal. The signal processing unit may include an A/D converter as a device that performs the conversion. The digital signal obtained by the conversion performed by the signal processing unit can be transmitted to the information processing unit. The digital signal can be handled as data related to light (hereinafter, also referred to as "light data") by the information processing unit. The light data may be light data including fluorescence data, for example. More specifically, the light data may be data of light intensity, and the light intensity may be light intensity data of light including fluorescence (the light intensity data may include feature quantities such as area, height, and width).

### (Information Processing Unit)

The information processing unit 6103 includes a processing unit that performs processing of various kinds of data (light data, for example), and a storage unit that stores various kinds of data, for example. In a case where the processing unit acquires the light data corresponding to a fluorescent dye from the detection unit, the processing unit can perform fluorescence leakage correction (a compensation process) on the light intensity data. In the case of a spectral flow cytometer, the processing unit also performs a fluorescence separation process on the light data, and acquires the light intensity data corresponding to the fluorescent dye. The fluorescence separation process may be performed by an unmixing method disclosed in JP 2011-232259 A, for example. In a case where the detection unit includes an image sensor, the processing unit may acquire morphological information about the biological particles, on the basis of an image acquired by the image sensor. The storage unit may be designed to be capable of storing the acquired light data. The storage unit may be designed to be capable of further storing spectral reference data to be used in the unmixing process.

In a case where the biological sample analyzer includes the sorting unit described later, the information processing unit can determine whether to sort the biological particles, on the basis of the light data and/or the morphological information. The information processing unit then controls the sorting unit on the basis of the result of the determination, and the biological particles can be sorted by the sorting unit.

The information processing unit may be designed to be capable of outputting various kinds of data (such as light data and images, for example). For example, the information processing unit can output various kinds of data (such as a two-dimensional plot or a spectrum plot, for example) generated on the basis of the light data. The information processing unit may also be designed to be capable of accepting inputs of various kinds of data, and accepts a gating process on a plot by a user, for example. The information processing unit may include an output unit (such as a display, for example) or a user interface (such as a keyboard, for example) for performing the output or the input.

The information processing unit may be designed as a general-purpose computer, and may be designed as an information processing unit that includes a CPU, a RAM, and a ROM, for example. The information processing unit may be included in the housing in which the light irradiation unit and the detection unit are included, or may be located outside the housing. Further, the various processes or functions to be executed by the information processing unit may be realized by a server computer or a cloud connected via a network.

### (Sorting Unit)

The sorting unit 6104 can perform sorting of biological particles, in accordance with the result of determination performed by, for example, the information processing unit. The sorting method may be a method by which droplets containing biological particles are generated by vibration, electric charges are applied to the droplets to be sorted, and the traveling direction of the droplets is controlled by an electrode. The sorting method may be a method for sorting by controlling the traveling direction of biological particles in the flow channel structure. The flow channel structure has a control mechanism based on pressure (injection or suction) or electric charge, for example. An example of the flow channel structure may be a chip (the chip disclosed in JP 2020-76736 A, for example) that has a flow channel structure in which the flow channel C branches into a recovery flow channel and a waste liquid flow channel on the downstream side, and specific biological particles are collected in the recovery flow channel.

### 2. Second Embodiment (Cell Treatment Method)

Fig. 9 illustrates an example of a flow chart of a cell treatment of the present technology. As illustrated in Fig. 9, the treatment method of the present technology may include a sample preparation step S100, a step S110 of acquiring information regarding the cells before treatment, an estimation step S120, a treatment step S130, and a step S140 of acquiring information regarding the cells after treatment. These steps will be described below.

### (1-1) Sample Preparation Step

In the sample preparation step, the cell culture unit 1 prepares a cell group containing a target cell. The type of the target cell is not particularly limited, and may be any of human-derived cells, immune cells, animal-derived cells, plant-derived cells, microorganism-derived cells, cancer cells, normal cells, stem cells, epithelial cells, and organoids. For example, in the case of animal cells, examples of the target cells include cells in blood and cells collected from biological tissues, and the like. Furthermore, the target cell may be either an adherent cell or a non-adherent cell. Moreover, the cell group may be peripheral blood mononuclear cells (PBMCs) collected from a patient or a donor. A cell group containing cells that attack specific cancer cells is produced by culturing and expanding the collected PBMCs and/or gene introduction, and the cell group is used for therapeutic use, for example, as a cell preparation. The PBMCs include various immune cell groups, and include T cells, B cells, macrophages, and the like. The T cells may further include a T cell subset including naive T cells, central memory T cells, effector T cells, and the like.

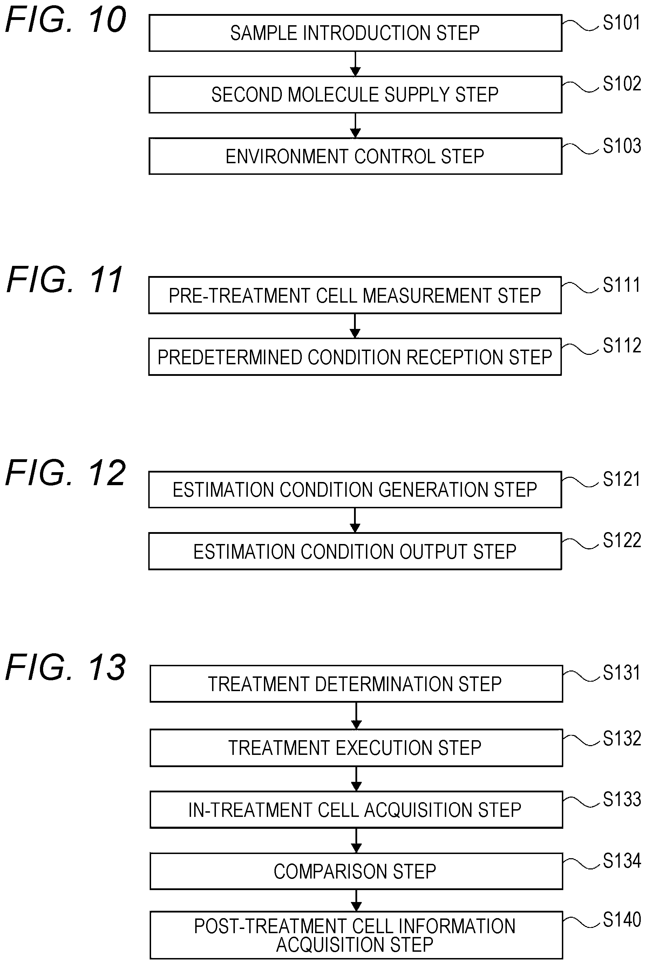

An example of a flowchart of the sample preparation step of the present technology is illustrated in Fig. 10. As illustrated in Fig. 10, the sample preparation step S100 may include a sample introduction step S101. In the sample introduction step, the cell group containing the target cell introduced from the cell introduction unit 106 of the cell culture unit 1 is captured in the culture vessel 101 by the first molecule capable of binding to the cell. In this step, the non-target cells may be recovered from the waste liquid storage unit 112 or the cell recovery unit 113 without being captured in the culture vessel 101.

The sample preparation step S100 may include a second molecule supply step. In the second molecule supplying step, the target cell supplemented to the culture vessel 101 can be bound to the second molecule supplied from the second molecule supplying unit. The supplied second molecule may be, for example, a fluorescent reagent, so that the measurement unit 3 can discriminate the target cell. Note that the target cell which is not bound to the second molecule and is not discriminated as the target cell by the measurement unit 3 may be treated in a treatment step described later and collected from the waste liquid storage unit 112 or the cell recovery unit 113. Without using the second molecule, a cell may be identified on the basis of an image that is identified from information learned from a bright field image, a phase difference image, a polarized image, and a non-stained image and a fluorescence image and is pseudo-stained for each feature of the cell, and the cell may be released by light stimulus. The identification may be performed by the information processing unit 2. The information processing unit 2 drives the optical control unit 210 on the basis of a result of the identification. The optical control unit 210 may execute the light stimulus.

The sample preparation step S100 may include an environment control step. In the environment control step, the environment of the culture vessel 101 containing cells before treatment is controlled by the environment control unit. For example, the culture vessel 101 may be supplied with a physicochemical environment such as humidity, pH, osmotic pressure, oxygen partial pressure, and carbon dioxide partial pressure from the physicochemical environment supply unit 114 under the control of the physicochemical environment by the environment control unit. Alternatively, for example, the culture vessel 101 may be supplied with a physiological environment such as a stimulating factor, a culture solution, a hormone, a cytokine, and an interleukin from at least one of the activator supply unit 108, the gene supply unit 109, or the culture solution supply unit 110 under the control of the physiological environment by the environment control unit. Thus, the optimum environment for the target cell in the culture environment is provided, and the survival rate and/or the culture efficiency of the cell can be enhanced. Thus, an optimum physiological environment is provided to the target cell in the culture environment, and the culture efficiency of the cell can be improved. Note that The environmental control step may be appropriately executed not only in the sample preparation step but also in the preceding and subsequent steps.

### (1-2) Step of Acquiring Information Regarding Cell Before Treatment

In the step of acquiring information regarding the cells before treatment, the information processing unit 2 acquires information regarding the target cells before treatment prepared in the sample preparation step. Fig. 11 illustrates an example of a flowchart of the step of acquiring information regarding the cells before treatment according to the present technology. As illustrated in Fig. 11, the step S110 of acquiring information regarding the cells before treatment may include a cell measurement step before treatment. In the cell measurement step before the treatment, the measurement unit 3 (in particular, the image acquisition unit and/or the signal detection unit) acquires image information and/or signal information. From the acquired image information and/or signal information, it is possible to extract information regarding the target cell before treatment. The image information and/or the signal information may be information derived from the second molecule, for example, the fluorescent reagent imparted in the sample preparation step.

The information regarding the cell before the treatment extracted by the measurement unit 3 may be, for example, at least one of a cell configuration, a form, a state, a number, a proportion, a type, an increase rate, a survival rate, genetic information, or information regarding a molecule. The information regarding the molecule includes, but is not particularly limited to, a gene expression control factor such as a transcription factor or a transcription control factor, information regarding a molecular marker, information regarding a cell surface antigen, sequence information, molecular weight, type, and the like. The information regarding the cell may be acquired by the measurement unit 3 and then transmitted to the estimation unit 300. Furthermore, the information regarding the cell may be transmitted to the database 500 of the information processing unit 2, and the database 500 may be updated.

The step S110 of acquiring information regarding the cells before treatment may include a predetermined condition reception step. In the predetermined condition reception step, the information processing unit 2 may include a step of receiving information regarding a predetermined treatment condition or a target cell after predetermined treatment. The predetermined treatment condition and the information regarding the target cell after the predetermined treatment may be received by a user interface appropriately set in the information processing unit. Thus, in the estimation step described later, it is possible to estimate the information regarding the cell after the desired processing by the user and the treatment condition for obtaining the desired treatment condition. Furthermore, a threshold value may be obtained from the database 500 and/or an external database for the information regarding the predetermined treatment condition and the target cell after the predetermined treatment. Thus, in the treatment step described later, for example, in a case where it is determined that the number of cells does not satisfy the threshold value and is insufficient, it is possible to determine that the initial cells are recollected or the production is stopped again, and in a case where the number of cells satisfies the threshold value, it is possible to determine that the cell treatment is continued.

In the predetermined condition reception step, the information processing unit 2 may further include a step of receiving information regarding approval of the cell commercialized as a threshold value from the database 500 and/or the external database. Thus, in the estimation step described later, by referring to the information regarding the approval of the existing commercialized cell, it is possible to present the treatment condition of the cell and the information regarding the cell necessary for satisfying the approval criteria to the user. The information regarding approval of the cell can be appropriately selected from information regarding the cell, treatment conditions of the cell, and information regarding therapy. Note that the information regarding the approval of the cell can be received by the user interface appropriately set in the information processing unit 2. The estimation unit may set the information regarding the predetermined cell and/or the predetermined treatment condition on the basis of the information regarding the approval of the cell.

The treatment condition may include a culture condition related to at least one of a stimulating factor or a number of culture days. In addition, the specified predetermined treatment condition may be cell sorting or cell culture control to be processed by the control unit 200 as described later.

The information regarding the target cell after treatment may be information regarding therapy of the cell in addition to the information regarding the cell before treatment described above. The information regarding therapy may be a therapeutic effect, a response rate, a recurrence rate, a side effect, and a treatment history of the patient of the cells. Thus, in a learning step to be described later, the information regarding the cell after treatment for reaching the information regarding therapy is learned, and a cell configuration and a treatment according to the individual patient can be executed.

The predetermined treatment condition received by the information processing unit 2 or the information regarding the target cell after the predetermined treatment may be appropriately transmitted to the estimation unit 300 and/or the database 500.

### (1-3) Estimation Step

In the estimation step, the estimation unit 300 estimates, on the basis of information regarding the target cell before treatment acquired in the cell measurement step before treatment and at least one of a predetermined treatment condition acquired in the predetermined condition reception step or the information regarding the target cell after predetermined treatment, a treatment condition under which the cell group is derivable to information regarding the predetermined cell or information regarding the cell after treatment of the cell group derived under the predetermined treatment condition as an estimation condition. In the present description, the estimation condition may be "a treatment condition under which the cell group is derivable to information regarding the predetermined cell" generated by the estimation processing by the estimation unit, or "information regarding the cell after treatment of the cell group derived under the predetermined treatment condition" generated by the estimation processing by the estimation unit. That is, the estimation condition may mean the estimated treatment condition or information regarding the estimated cell.

The treatment condition under which the cell group is derivable to information regarding the predetermined cell is a treatment condition that is derivable by the control unit 200 to reach the information regarding the predetermined cell designated in the predetermined condition reception step, and may be, for example, a cell sorting or a culture condition as described later.

The information regarding the cell after treatment of the cell group derived under the predetermined treatment condition is information regarding the cell after treatment that can be acquired by the control unit 200 executing predetermined treatment designated by the user or the like. The predetermined treatment designated by the user or the like is not particularly limited as long as it is a treatment condition in a treatment step to be described later. The information regarding the cell after treatment is not particularly limited as long as it is as described above. For example, the information regarding the cell after treatment may include at least one of a configuration, a state, a number, a proportion, an increase rate, a survival rate, a response rate, a recurrence rate, or a side effect of the cell after treatment.

Fig. 12 illustrates an example of a flowchart of the estimation step of the present technology. As illustrated in Fig. 12, the estimation step S120 may include an estimation condition generation step. In the estimation condition generation step, the estimation unit 300 generates, on the basis of the information regarding the target cell before treatment acquired in the cell measurement step before treatment and the predetermined treatment condition acquired in the predetermined condition reception step, information regarding the cell after treatment of the cell group. Alternatively, the estimation unit 300 generates, on the basis of the information regarding the target cell before treatment acquired in the cell measurement step before treatment and the information regarding the target cell after predetermined treatment acquired in the predetermined condition reception step, a treatment condition under which the cell group is derivable to information regarding the predetermined cell.

The estimation unit 300 may acquire the information regarding the target cell before treatment from the measurement unit 3 and/or the database 500. Furthermore, the estimation unit 300 may acquire information regarding the cell after treatment desired by the user and the desired treatment condition from at least one of the database 500, the external database, or the user interface of the information processing unit 2.

In the estimation condition generation step, the estimation unit 300 may further generate an inference condition using any one of a first learner and a second learner generated by the learning unit 400 in a learning step to be described later.

In the present description, the estimation step means a step of executing processing of generating the derivable treatment condition or the information regarding the cell after treatment on the basis of the information regarding the target cell or the like, that is, the estimation step includes inference processing in the machine learning field. That is, in the present description, the estimation step may be referred to as an "inference step". Furthermore, in the present description, the estimation condition may be referred to as an "inference condition".

The estimation step S120 may include an estimation condition output step. In the estimation condition output step, the estimation unit 300 outputs the estimation condition generated in the estimation condition generation step. The output condition is not particularly limited as long as it is the estimation condition generated in the estimation condition generation step described above. The estimation condition may be presented to the user via the user interface appropriately set in the information processing unit 2. Thus, the user can visually recognize the output condition.

The output estimation condition may be transmitted to the database 500, and the database 500 may be updated. Furthermore, the output estimation condition may be transmitted to the control unit 200 of the cell culture unit 1, and the control unit 200 can perform treatment of the target cell inside the cell culture unit 1 on the basis of the output condition.

### (1-4) Treatment Step

In the treatment step, the control unit performs treatment related to the sample containing the cell on the basis of the information regarding the cell acquired in the step of acquiring information regarding the cells before treatment and the estimation condition output in the estimation step. In the treatment related to the sample containing the cells, for example, cell sorting or culture control may be performed on the cells before treatment supplemented to the cell culture apparatus.

The cell sorting is, for example, a step of stimulating the degradable linker by optical control by the optical control unit to release the cells bound to the sample holding unit 100 via the degradable linker. It is possible to discriminate between cells that can be treated and cells other than the target on the basis of the information regarding the cells acquired in the step of acquiring information regarding the cells before treatment, for example, a fluorescence signal derived from the second molecule imparted to the cells, and to release only the cells other than the target by the stimulus. Without using the second molecule, a cell may be identified on the basis of an image that is identified from information learned from a bright field image, a phase difference image, a polarized image, and a non-stained image and a fluorescence image and is pseudo-stained for each feature of the cell, and the cell may be released by light stimulus. The identification may be performed by the information processing unit 2. The information processing unit 2 can drive the optical control unit on the basis of a result of the identification. The optical control unit may execute light stimulus.

The culture control is, for example, a step of culturing a sample containing cells bound to the sample holding unit 100 under the environmental control by the environmental control unit. For example, the culture vessel 101 may be subjected to feedback control by the environment control unit on the basis of the information regarding the cells acquired in the step of acquiring information regarding the cells before treatment, and may be supplied with a physicochemical environment, such as humidity, pH, osmotic pressure, oxygen partial pressure, and carbon dioxide partial pressure from the physicochemical environment supply unit 114. Alternatively, for example, the culture vessel 101 may be subjected to the feedback control by the environment control unit on the basis of the information regarding the cells acquired in the step of acquiring information regarding the cells before treatment, and a physiological environment, such as a stimulating factor, a transcription factor, a transcription control factor, a culture solution, a hormone, a cytokine, and an interleukin may be supplied from at least one of the activator supply unit 108, the gene supply unit 109, or the culture solution supply unit 110. Thus, the optimum environment for the target cell in the culture environment is provided, and the survival rate and/or the culture efficiency of the cell can be enhanced.

An example of the feedback control in the step of acquiring information regarding the cells before treatment will be described below. For example, in a case where treatment in the cell treatment system 1000 is executed using PBMC as an initial cell group before culture treatment, proliferation of CD4 and CD8 positive cells contained in the cell group after the culture treatment may differ depending on the type of culture solution, the type and concentration of the stimulating molecule, and the number of days of culture. Furthermore, the ratio of naive T cells, central memory T cells, and effector cells included in the cell group, that is, the ratio of T cell subsets may also be different. In a case where the anti-CD3 antibody is added to the cell group described above from the activator supply unit 108 in the cell treatment system 1000, the CD8 positive cells proliferate significantly, whereas in a case where the anti-CD3/CD28 antibody is added, the cells can proliferate while the ratio of CD4 and CD8 positive cells is maintained. Moreover, the proportion of T cell subsets after culture for a certain number of days may vary depending on the culture solution, cytokine, and/or cytokine type and concentration.

For example, in the feedback control in the step of acquiring information regarding the cells before treatment, the environment control unit can control the activator supply unit 108 to supply only the anti-CD3 antibody into the culture vessel in the initial culture step. This increases the proportion of CD8 positive cells in the cell group. Then, in response to the determination by the measurement unit 3 that the ratio of CD8 positive cells has reached the target value, the environment control unit may control the activator supply unit 108 so as to stop the supply of the anti-CD3 antibody by the activator supply unit 108.

As described above, the environment control unit can control the activator supply unit 108 to supply the activator into the culture vessel so as to increase or decrease the ratio of one or more predetermined cells in the cell group. In addition, the environment control unit can control the activator supply unit 108 to supply the activator into the culture vessel so as to increase or decrease the survival rate of one or more predetermined cells in the cell group. Moreover, the environment control unit can control the activator supply unit 108 to supply the activator into the culture vessel so as to increase or decrease gene introduction efficiency of one or more predetermined cells in the cell group.

Furthermore, the environment control unit may control the activator supply unit 108 to additionally supply the anti-CD28 antibody into the culture vessel. This makes it possible to cause the cell group to proliferate while maintaining the ratio of CD4 and CD8 positive cells in the cell group. Moreover, in response to the measurement unit 3 determining that the proportion of the T cell subset in the cell group has reached the goal, the environment control unit may control the activator supply unit 108 so as to change the type and/or concentration of the culture solution and cytokine supplied by the activator supply unit 108.

As described above, the environment control unit can control the activator supply unit 108 to supply the activator into the culture vessel so as to maintain the ratio of one or more predetermined cells in the cell group.

The treatment conditions processed by the cell sorting and/or culture control may be transmitted to the database 500, and the database 500 may be updated. This improves the accuracy of generation of the estimation condition in the estimation condition generation step. Note that the contents processed by the cell sorting and/or culture control may be stored with, for example, a date and a period such as the number of culture days, the culture solution replenishment, or the replacement date in addition to the actual treatment conditions.

Fig. 13 illustrates an example of a flowchart of the treatment step of the present technology. As illustrated in Fig. 13, the treatment step S130 may include a treatment determination step S131. In the treatment determination step S131, the control unit executes treatment determination on the basis of the predetermined condition acquired in the predetermined condition reception step and the estimation condition output in the estimation step. The predetermined condition and/or the estimation condition may be further presented from the user interface of the information processing unit 2 that can be input by the user. The treatment condition of the subsequent stage can be executed on the basis of the predetermined condition and/or the estimation condition selected by the user. For example, an index that allows the user to determine whether or not to proceed to a subsequent treatment condition such as cell culture or cell sorting may be presented on the user interface. Thus, the user can set the treatment condition via the user interface as necessary, and the information processing unit can execute the designated processing.

For example, in a case where the estimation condition satisfies a predetermined condition, the control unit 200 can proceed to a subsequent treatment execution step (for example, a culture step), but in a case where the estimation condition does not satisfy the predetermined condition, it is possible to appropriately select whether to remove unnecessary cells by the selection step, a production stopping step to control the control unit to stop the production of cells, or whether to control the control unit to re-collect cells. Thus, it is possible to determine whether or not treatment can be executed before the treatment by the control unit 200 is started, and it is possible to stop the process before reaching the final step of the cell treatment system to improve productivity. In the production stopping step, the control unit may perform control to stop the culture of the cell group and deliver the cells to the waste liquid storage unit. In the re-collecting step, the control unit may control to continue the culture of the cell group and collect new cells from the cell introduction unit 106. Furthermore, in the treatment determination step, the user interface may be provided with an input unit that allows the user to select, for example, whether or not to proceed to cell culture or cell sorting in the subsequent stage.

The treatment step S130 may include the treatment execution step S132 (for example, a culture step). As described above, in the culture step, the control unit cultures a sample containing cells bound to the sample holding unit 100 under the environmental control by the environmental control unit.

The treatment step S130 may include the in-treatment cell information acquisition step S133 (for example, measurement step). In the in-treatment cell information acquisition step S133, the control unit controls the measurement unit 3 to start measurement, and acquires information regarding the cell in treatment. The measurement method by the measurement unit 3 is not particularly limited as long as it is as described above. By acquiring information regarding the cell over time, the cell can be always observed, and the abnormality of the cell can be immediately detected. The information about the cell during the processing is similar to the information about the cell before the treatment, and is not particularly limited. The information regarding the cell in treatment may be transmitted to the database 500, and the database 500 may be updated.

Hereinafter, an example of acquiring information regarding the cell in treatment in the in-treatment cell information acquisition step S133 will be described. For example, in a case where PBMCs are collected, the PBMCs can be measured by the measurement unit 3 including the image acquisition unit 600 and/or a signal detection unit 700. Lymphocyte fractionation is specified on the basis of forward scattered light and side/backward scattered light acquired by the measurement unit 3 described above, and the number and ratio of living cells can be specified from the types of cells such as T cells, B cells, and macrophages, T cell subsets such as naive T cells, central memory T cells, and effector T cells, and information regarding nuclear staining of the target cell from the information regarding fluorescence of the target cell stained with fluorescence and/or a metal-labeled antibody. In addition, the PBMC may be obtained from an image that is identified from information learned from the non-stained image and the fluorescence image and is pseudo-stained for each cell feature. Thus, since the cell information can be acquired during the treatment step, it is possible to omit a movement step of selecting and extracting cells from the culture vessel in the processing step and a measurement step after extraction. This makes it possible to move the cells from the culture vessel while maintaining a sterile state, and furthermore, it is possible to simplify the steps in the cell treatment system by omitting the measurement step in the measurement unit.

Note that, in the measurement step, it is possible to analyze the proliferation state of cells from the information regarding the size and number of cell masses acquired from the measurement unit 3. For example, in a case of T cells, a cell mass may be formed during cell proliferation. The number of the cells is estimated by measuring the area of the cell mass, and furthermore, the total number of cells in the vessel can be estimated by measuring the number of the cells. By acquiring the measured value related to the mass in time series, the proliferation state of the cell can be confirmed.

The treatment step S130 may include a comparison step S134. In the comparison step S134, the control unit 200 compares the information regarding the cell in treatment acquired in the in-treatment cell information acquisition step S133 with the predetermined condition acquired in the predetermined condition reception step, and executes treatment determination. For example, in a case where the information regarding the cell in treatment satisfies the predetermined condition, the control unit 200 can proceed to the culture step in the subsequent stage, but in a case where the predetermined condition is not satisfied, it is possible to appropriately select whether to remove unnecessary cells by the selection step, the production stopping step to control the control unit to stop the production of cells, or whether to control the control unit to re-collect cells. Thus, by observing the information regarding the cell in treatment from the measurement unit 3 over time, it is possible to switch the content of the treatment in the middle of the treatment and perform optimization processing.

### (1-5) Step of Acquiring Information Regarding Cells After Treatment

In the step S140 of acquiring information regarding the cells after treatment, the measurement unit 3 acquires information regarding the cells after treatment. In a case where the information regarding the cells after treatment reaches the predetermined value, the cell group is collected from the cell recovery unit. The recovered cells may be evaluated for information regarding therapy by the information processing unit or may be evaluated by an evaluation device outside the system. The evaluated information regarding therapy of the cells after treatment may be transmitted to the database 500 after the evaluation, and the database 500 may be updated.

As described above, the information regarding the target cell after treatment may be information regarding therapy of the cells in addition to the information regarding the cells before the treatment. The information regarding therapy may be a therapeutic effect, a response rate, a recurrence rate, a side effect, and a treatment history of the patient of the cells. Thus, in a learning step to be described later, the information regarding the cell after treatment for reaching the information regarding therapy is learned, and a cell configuration and a treatment according to the individual patient can be executed.

### (1-6) Learning Step

In the second embodiment, the cell treatment method according to the present technology may further include a learning step. In the learning step, the learning unit 400 learns the relevance between the information regarding the cell and the information regarding therapy of the cell and generates the first learner. Alternatively, the learning unit 400 learns the relevance between the information regarding the cell and the treatment condition and generates the second learner. Thus, it is possible to estimate the treatment conditions for achieving a desired cell group and the therapeutic effect of a desired cell from the information regarding the cell before treatment in the cell group, and the efficiency of the cell treatment process can be enhanced.

The estimation unit may execute the estimation processing described above using the generated first learner and/or second learner.

The first learner learns the relevance between the cell and the information regarding therapy using the information regarding the cell treated under the treatment condition estimated by the estimation unit 300 and the information regarding therapy of the cell as input information for learning. The information regarding the cell after treatment and the information regarding therapy of the cell after treatment are as described above. The estimation unit 300 may perform inference using a learner generated by the learner. Thus, it is also possible to predict the optimal cell configuration according to the patient.

For example, in a case where the cell group processed in the cell treatment system 1000 is a PBMC, the PBMC is used as a therapeutic application. It is also possible to optimize the timing of collecting PBMCs by learning the relevance between the information regarding the cell group after treatment such as the cell configuration, the number, and the condition of the PBMCs and the information regarding therapy such as donor information, the response rate of the treatment, the incidence rate of side effects, and the quality of the cells. Thus, it is possible to estimate the treatment conditions for achieving a desired cell group and the therapeutic effect of a desired cell from the information regarding the cell before treatment in the cell group, and the efficiency of the cell treatment process can be enhanced.

The second learner learns the relevance between the information regarding the cell before treatment and the treatment condition by using the information regarding the cell before treatment, the treatment condition estimated by the estimation unit 300, and the cell before treatment treated under the treatment condition as the input information for learning. The treatment condition is not particularly limited as long as it is a condition to be treated in the treatment step. Thus, it is possible to estimate the treatment conditions for achieving a desired cell group and the therapeutic effect of a desired cell from the information regarding the cell before treatment in the cell group, and the efficiency of the cell treatment process can be enhanced. The estimation unit 300 may perform inference using a learner generated by the learner. This makes it possible to estimate the treatment conditions and the therapeutic effect.

The first learner may be a learner generated by machine learning a data set including information regarding cells and information regarding therapy of the cells. The machine learning may be, for example, deep learning. The machine learning may be executed according to an information processing device or an information processing method described in WO 2021/049365, for example. In the machine learning, the information regarding the cell may be handled as an explanatory variable, and the information regarding therapy of the cell may be handled as an objective variable.

Furthermore, the second learner may be a learner generated by causing machine learning of a data set including the information regarding the cell before treatment, the treatment conditions, and information regarding the cell before treatment treated under the treatment conditions (that is, information regarding the cell after treatment). The machine learning may be, for example, deep learning. The machine learning may be executed according to an information processing device or an information processing method described in WO 2021/049365, for example.

In the machine learning, the information regarding the cell before treatment and the treatment condition may be handled as explanatory variables, and the information regarding the cell after treatment may be handled as an objective variable. Such handling of the explanatory variable and the objective variable may be applied, for example, in an embodiment in which the estimation unit estimates information regarding cells after processing of the cell group.

Alternatively, in the machine learning, the information regarding the cell before treatment and the information regarding the cell after treatment may be treated as explanatory variables, and the treatment condition may be handled as an objective variable. Such handling of the explanatory variable and the objective variable may be applied, for example, in an embodiment in which the estimation unit estimates the treatment condition for enabling the information regarding the predetermined cell to be derived from the cell group.

### 3. Configuration Example of Cell Treatment System

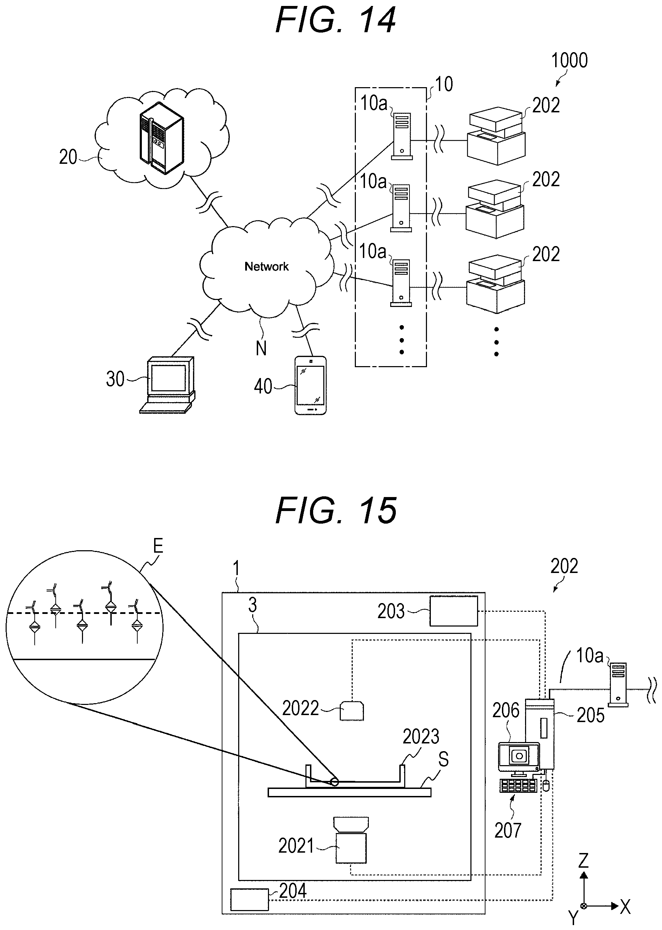

A configuration example in the embodiment of the cell treatment system according to the present technology will be described below with reference to Fig. 14 to 16.

Fig. 14 is a schematic diagram schematically illustrating a configuration example of the cell treatment system 1000 according to the present technology. For example, as illustrated in Fig. 14, the cell treatment system 1000 includes a terminal device 10, an information processing device 20, a first terminal 30, and a second terminal 40. Each of the terminal devices 10 is connected to an observation device 202 in a wired or wireless manner.

In the present embodiment, the terminal device 10, the information processing device 20 (corresponding to the information processing unit 2 described above), the first terminal 30, and the second terminal 40 are communicably connected to each other via the network N. The network N may be, for example, the Internet, a mobile communication network, a local area network, or the like, or may be a network in which a plurality of types of networks is combined.

### [Terminal Device]

As illustrated in Fig. 14, the terminal device 10 includes a plurality of gateway terminals 10a, and each of the gateway terminals 10a is connected to the observation device 202 in a wireless or wired manner via a control recording PC 205 (see Fig. 15). The observation device 202 is handled by a user of the cell treatment system.

Note that the cell treatment system 1000 of the present embodiment typically has a configuration in which a plurality of observation devices 202 (for example, the microscope system or the biological sample analyzer described above) is connected to the information processing device 20 via the terminal device 10 as illustrated in Fig. 14, but is not limited thereto, and may have a configuration in which a single observation device 202 is connected to the information processing device 20 via the terminal device 10.

A configuration example of the observation device 202 will be described with reference to Fig. 15. Note that X, Y, and Z axes illustrated in Fig. 15 are three axial directions orthogonal to each other.

The observation device 202 includes the cell culture unit 1 and the measurement unit 3 described in 1. and 2. described above. The cell culture unit 1 and the measurement unit 3 may be integrally configured, for example.

The cell culture unit 1 may be configured as an incubator for incubating cells. The incubator includes a physicochemical environment supply unit 203 and a detection unit 204. The cell culture unit 1 further includes a culture vessel group 2023. The physicochemical environment supply unit 203 is configured to control, for example, any one or more of humidity, temperature, and gas in the incubator.

The measurement unit 3 may be configured to be capable of observing cells in the incubator, and may be configured as, for example, a microscope system. In this case, the measurement unit 3 may include the image acquisition unit. In addition, the measurement unit 3 may be configured as, for example, a biological sample analyzer. In this case, the measurement unit may include the signal detection unit.

The above-described components included in the cell culture unit 1 and the measurement unit 3 are connected to the control recording computer (PC) 205. The control recording PC 205 is also connected to the display device 206 and the input unit 207.

The incubator accommodates the measurement unit 3, the physicochemical environment supply unit 203, and the detection unit 204. The incubator may be configured as a culture apparatus for culturing cells. The incubator has a function of adjusting the internal temperature, humidity, and the like to predetermined values by the contained physicochemical environment supply unit 203. The incubator is configured such that any gas can flow into the incubator by the physicochemical environment supply unit 203. The type of the gas is not particularly limited, and is, for example, nitrogen, oxygen, carbon dioxide, or the like.

The measurement unit 3 includes an imaging unit 2021 and a light source 2022. The imaging unit 2021 may be configured to be capable of imaging a cell accommodated in the culture vessel 2023 (dish) and generate an observation image of the cell, or is configured to be capable of acquiring a signal from the cell. As illustrated in E of Fig. 15, the first molecule described in (3-1) above may be immobilized on the culture vessel 2023. The description in the (3-1) above also applies to the culture vessel 2023.

The imaging unit 2021 includes a lens barrel including a lens group movable in an optical axis direction (Z-axis direction), a solid-state imaging element such as a complementary metal oxide semiconductor (CMOS) or a charge coupled device (CCD) that images subject light passing through the lens barrel, a drive circuit that drives these elements, and the like.

The imaging unit 2021 is configured to be movable in an optical axis direction (Z-axis direction) and a horizontal direction (a direction orthogonal to the Z-axis direction), and images the cell accommodated in the culture vessel 2023 while moving in the horizontal direction. Furthermore, the imaging unit 2021 may be configured to be capable of capturing not only a still image but also a moving image.

The imaging unit 2021 according to the present embodiment is, for example, a visible light camera, but is not limited thereto, and may be an infrared (IR) camera, a polarization camera, or the like.

The light source 2022 irradiates the culture vessel 2023 with light when the cell in the culture vessel 2023 is imaged by the imaging unit 2021. As the light source 2022, for example, a light emitting diode (LED) or the like that emits light of a specific wavelength is adopted. In a case where the light source 2022 is an LED, for example, a red LED that emits light having a wavelength of 640 nm is adopted.

As illustrated in Fig. 15, one culture vessel 2023 may be installed on the stage S, or a plurality of culture vessels may be installed. The culture vessel 2023 can be disposed between the imaging unit 2021 and the light source 2022. An observation stage S is configured to transmit light emitted from the light source 2022. Note that the light source 2022 may be disposed so as to irradiate the inside of the vessel 2023 with light from the imaging unit 2021 side.

A material constituting culture vessel is not particularly limited, but culture vessel 2023 is formed by, for example, an inorganic material such as glass or silicon, or an organic material such as a polystyrene resin, a polyethylene resin, a polypropylene resin, an ABS resin, nylon, an acrylic resin, a fluororesin, a polycarbonate resin, a polyurethane resin, a methylpentene resin, a phenol resin, a melamine resin, an epoxy resin, or a vinyl chloride resin, and is a transparent body through which light emitted from light source 2022 passes. Alternatively, in the culture vessel 23, a portion other than the portion through which light emitted from the light source 2022 passes may be formed by the materials listed above, or may be formed by a metal material.

The physicochemical environment supply unit 203 controls the temperature and humidity in the incubator and the gas introduced into the incubator, and creates an environment suitable for culturing cells. The physicochemical environment supply unit 203 can control the temperature in the incubator to, for example, any temperature of 20°C to 50°C.

The detection unit 204 is connected to the control recording PC 205 in a wireless or wired manner, and is configured to detect the temperature and the atmospheric pressure in the incubator, the illuminance and the oxygen concentration of the light source 2022, and the like, and output them to the control recording PC 205. The detection unit 204 is, for example, a solar panel type or battery type Internet of Things (IoT) sensor or the like, and the type thereof is not limited.

The control recording PC 205 is connected to the imaging unit 2021, the light source 2022, the physicochemical environment supply unit 203, the detection unit 204, and the gateway terminal 10a. The control recording PC 205 is configured to be capable of controlling the culture environment of the cell by controlling the imaging unit 2021, the light source 2022, the detection unit 204, and the physicochemical environment supply unit 203 on the basis of the outputs thereof.

For example, the control recording PC 205 is configured to be capable of storing the culture environment information output from the detection unit 204, and transmitting the culture environment information to the gateway terminal 10a. Here, the culture environment information of the present embodiment is, for example, information related to the pH of the culture solution, and the temperature, humidity, and oxygen concentration in the incubator, and has the same meaning in the following description.

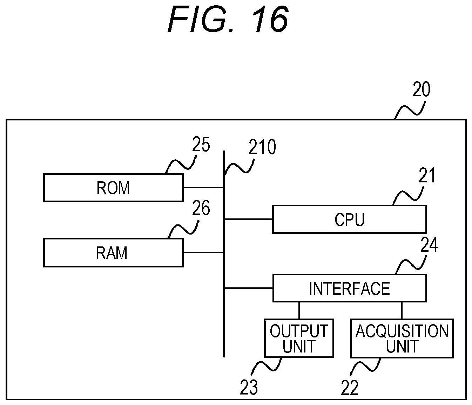

Upon receiving the culture environment information, gateway terminal 10a transmits, as the culture environment information, information regarding at least one of the pH of the culture solution or the temperature, humidity, or oxygen concentration in the incubator to acquisition unit 24 (see Fig. 16) via network N. The acquisition unit 24 outputs the acquired culture environment information to the storage unit 28 (see Fig. 16), and the culture environment information is stored in the storage unit 28.

In addition, the control recording PC 205 stores information regarding cells, culture environment information, information regarding the culture vessel 2023a, various information described in the above 1. and 2., or the like. The control recording PC 205 is configured to be capable of transmitting these pieces of information to the gateway terminal 10a.

The display device 206 is configured to be capable of displaying an observation image captured by the imaging unit 2021, information regarding a cell, culture environment information, or various types of information described in the above 1. and 2. The display device 206 is, for example, a display device using liquid crystal, organic electro-luminescence (EL), or the like.

The input unit 207 is an operation device such as a keyboard or a mouse for inputting an operation by the user. The input unit 207 according to the present embodiment may be a touch panel or the like integrally configured with the display device 206.

As illustrated in Fig. 14, from the viewpoint of improving the analysis accuracy of the information processing device 50, the terminal device 10 of the present embodiment is preferably configured by a plurality of gateway terminals 10a, but may be configured by a single gateway terminal 10a. In this case, the single gateway terminal 10a may be connected to the plurality of observation devices 202 in a wireless or wired manner via the control recording PC 205.

In addition, the gateway terminal 10a is typically a general-purpose gateway configured to be capable of mutually converting different protocols and address systems, but is not limited thereto, and may be a personal computer (PC) or the like set to serve as a gateway.

### [Information Processing Device]

Fig. 16 is a block diagram of the information processing device 20 according to the present embodiment. As illustrated in the drawing, the information processing device 20 can include a central processing unit (CPU) 21. The information processing (for example, the estimation step and/or the learning step) described above can be executed by the CPU 21. The CPUD 21 is configured to execute the functions of the estimation unit 300, the learning unit 400, and the database 500 described in 1. and 2. above.

Furthermore, the information processing device may also include the database 500. The information processing device 20 further includes hardware necessary for a computer, such as a read only memory (ROM) 25 and a random access memory (RAM) 26. The database 500 may exist, for example, in the ROM 25 or the RAM 26.

The CPU 21 loads a program for causing the information processing device 20 to execute information processing according to the present technology stored in, for example, the ROM 25 into the RAM 26 and executes the program. Thus, each block operation of the information processing device 20 described later is controlled.

The ROM 25 is a memory device in which various data, programs, and the like used in the information processing device 20 are fixedly stored. For example, the ROM 25 may store a program executed by the CPU 21.

The RAM 26 is a memory element such as a static random access memory (SRAM) used as a work area for the CPU 21, a temporary storage space of history data, and the like. The RAM 26 can be used as a work memory or the like when the CPU 21 executes processing.

The program is installed in the information processing device 20 via, for example, various storage media (internal memories). Alternatively, the program may be installed via the Internet or the like. The information processing device 20 of the present embodiment is a web server for performing quality evaluation of the fertilized egg F by cloud computing, but is not limited thereto, and for example, any other computer such as a PC may be used.

The information processing device 20 further includes an acquisition unit 22, an output unit 22, an I/O interface 24, and a bus 210.

The acquisition unit 22 acquires one or more observation images in which a cell or a cell population associated with unique identification information is imaged from a plurality of gateway terminals 10a (terminal devices 10) via the network N.

The output unit 23 outputs information generated according to the present technology to, for example, a computer via the network N.

The information processing device 20 may further include a non-volatile memory such as a hard disc drive (HDD) and a solid state drive (SSD). Thus, input information input from the terminal device 10, the first terminal 30, and the second terminal 40, a result of processing by the analysis unit 25, and the like can be stored.

The I/O interface 24 is communicably connected to the terminal device 10 and the first and second terminals 30 and 40 via the network N, and includes an acquisition unit 22 and an output unit 23. The I/O interface 24 functions as an input/output interface between the terminal device 10 and the first and second terminals 30 and 40.

The bus 210 is a signal transmission path for inputting and outputting various signals between the respective units of the information processing device 20. The CPU 21, the ROM 25, the RAM 26, and the I/O interface 29 described above are mutually connected through a bus 210.

Note that the configuration and function of the information processing device 20 are not limited to those described above.

### [First terminal]

The first terminal 30 includes a reception unit that receives information output from the output unit 23 or the second terminal 40 via the network N, an input unit that receives an input from a user of the cell treatment system of the present technology, and a transmission unit that transmits the information input via the input unit and the information received by the reception unit.

The first terminal 30 is typically a computer such as a laptop PC or a desktop PC, but is not limited thereto, and may be, for example, a smart device or a tablet terminal.

### [Second terminal]

The second terminal 40 includes a reception unit that receives information output from the output unit 23 or the first terminal 30 via the network N, an input unit that receives an input from a user of the cell treatment system of the present technology, and a transmission unit that transmits the information input via the input unit and the information received by the reception unit.

The second terminal 40 is typically a smart device, a tablet terminal, or the like, but is not limited thereto, and may be any other computer such as a laptop PC or a desktop PC, for example.

The first terminal 30 or the second terminal 40 may control the observation device 202 to execute the cell treatment according to the present technology.

### 4. Example

The cell samples were cultured under various treatment conditions, and the relevance between the treatment conditions and the cultured cells was analyzed. The experimental protocol and analysis results of the culture and analysis are described below.

### (1-1) Culture

Cell-containing samples were cultured using 12/24 well plates. As the sample, a peripheral blood mononuclear cell fraction derived from a healthy person or a peripheral blood mononuclear cell fraction derived from a patient was used. Materials and reagents used in the culture are as follows.

Peripheral blood mononuclear cells (PBMC): Characterized PBMC
Plasma: donor-derived plasma
Medium: TCell Expansion Medium
Antibody: Human CD3/CD28 T Cell Activator and Purified anti-human CD3 Antibody (clone: OKT3)
Cytokines: Human Recombinant IL-2, Human Recombinant IL-7, and Human Recombinant IL-15
PBS(-):DPBS, no calcium, no magnesium

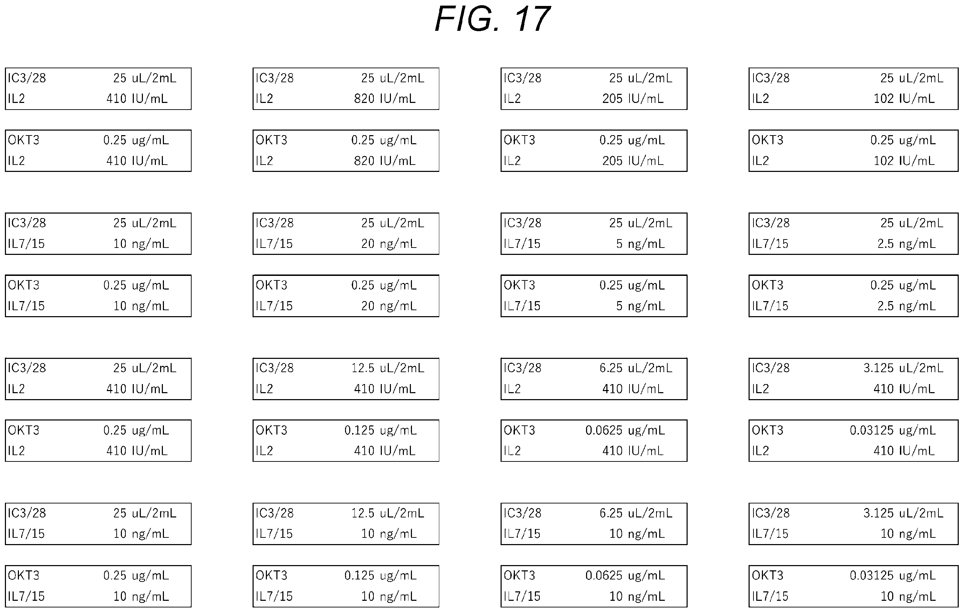

Media with and without 0.5% plasma were created. PBMC were placed in each well of the plate so as to be about 5 × 10⁵ cells/well or more. To each well, antibodies and cytokines were added at different concentrations. The antibodies and cytokines added at Day 0 and their added amounts are illustrated in Fig. 17. Each of the quadrangles illustrated in the drawing corresponds to each well. That is, a total of 32 stimulation patterns were used. For example, in the first column and the first row of the drawing, a medium to which IC 3/28 of 25 µL/2 mL and IL2 of 410 IU/mL are added is illustrated.

Antibodies and cytokines indicated by abbreviations in the drawing are as follows.
IC3/28:ImmunoCult Human CD3/CD28 T Cell Activator
IL2:Human Recombinant IL-2
IL7:Human Recombinant IL-7
IL15:Human Recombinant IL-15
OKT3:Purified anti-human CD3 Antibody(clone:OKT3)

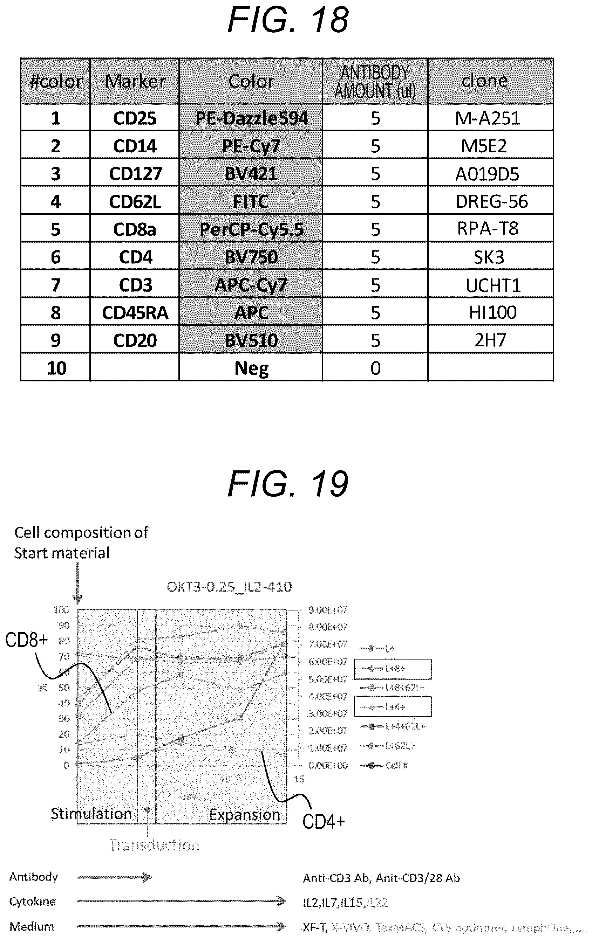

Cultures in each well were expanded every 3 or 4 days and cultured for a total of 14 days. In the expansion, specifically, the culture in each well was divided into two, and the same amount of medium as the divided amount was added to the divided culture solution. After the division, cytokines were added so that the initial concentration was maintained. No antibody was added after the division. That is, the antibody concentration was diluted 2-fold for each expansion. Furthermore, when the medium replacement is performed on day 3-4 or later, the antibody amount is 0. At the time of expansion treatment, cells were extracted, and the extracted cells were measured by flow cytometry. The measurement was performed using a commercially available flow cytometer. The staining antibody used in the measurement is illustrated in Fig. 18. Cells in the culture were stained by a mixture of nine staining antibodies of 1 to 9 in the drawing. In the drawing, the capture target, dye, amount, and clone name of each antibody are illustrated in the Marker column, Color column, antibody amount (ul) column, and clone column, respectively. Furthermore, as a negative control, a culture that was not stained with a staining antibody was also used. As analysis software, FlowJo (BD) was used.

Fig. 19 illustrates an overview of the experiment and measurement/analysis. The graph in the drawing illustrates the measurement result by the flow cytometer of one of the well groups (OKT3-0.25 pg/mL, IL2-410 IU/mL). The left Y-axis of the graph indicates the proportion (%) of cells, and the right Y-axis indicates the number of cells. The X-axis represents the day. The graph illustrates the variation of the proportion (or number) of each of the seven types of cells in the total target cells to be measured in the culture solution. A plot in the graph is a measurement result by the flow cytometer. In the graph, for example, there are plots on Day 0 (culture start date), Day 4, Day 7, Day 11, and Day 14, that is, the cells were expanded on these days, and a sample to be measured by the flow cytometer was acquired at the time of the expansion. As illustrated in the graph, for example, the proportion of the CD4-positive cell population did not change throughout the culture period (CD4+ in the drawing), but the proportion of the CD8-positive cell population greatly increased from Day 0 to Day 4 after the start of culture (CD8+ in the drawing). Thus, the proportion (or number) of cell types identified by the context of expression of a given marker was recorded over the culture period.

In addition, the lower part of the drawing illustrates the stimulation applied to the culture solution. For example, stimulation with the antibody continues until the first expansion has taken place, that is, until day 4. On the other hand, the stimulation by the cytokine and the stimulation by the medium are continued for a culture period of 14 days.

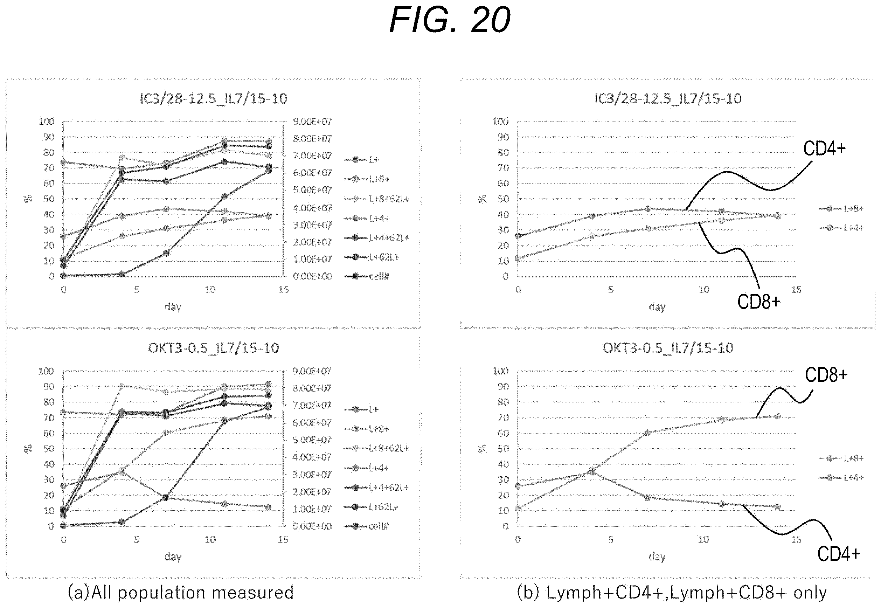

Measurement data for a part of the well group is illustrated in Fig. 20. As illustrated in the drawing, it can be seen that there are a cell group that is easy to proliferate and a cell group that is difficult to proliferate depending on the culture conditions. Note that, in the culture conditions, the difference in proliferation of CD4+ cells and CD8+ cells was large. For example, as illustrated in the upper right part of the drawing, in the medium to which IC 3/28 and IL7 and IL15 were added, CD4+ cells and CD8+ cells proliferated similarly. On the other hand, as illustrated in the lower right of the drawing, in the medium to which OKT3, IL7, and IL15 were added, CD8+ cells proliferated greatly, but the degree of proliferation of CD4+ cells was small.

### (1-2) Creation of Data Set

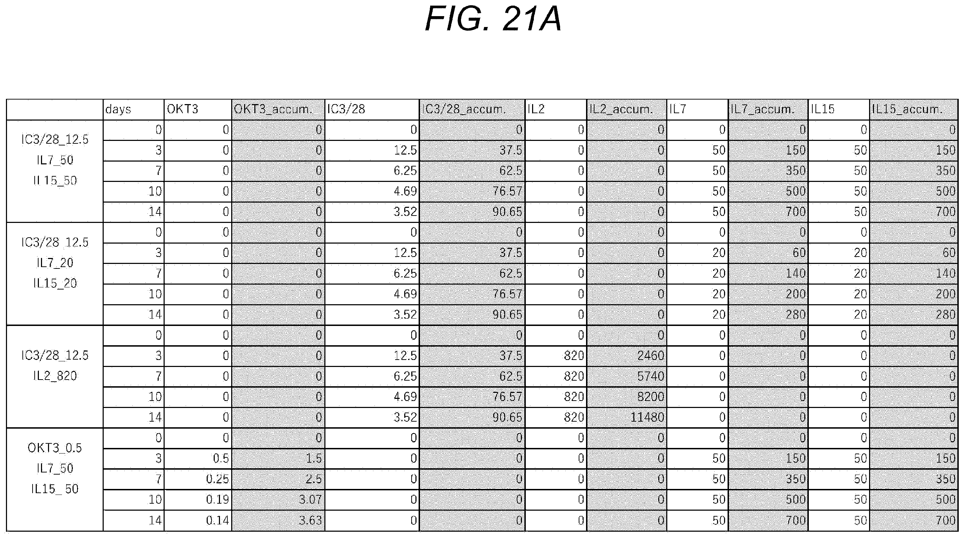

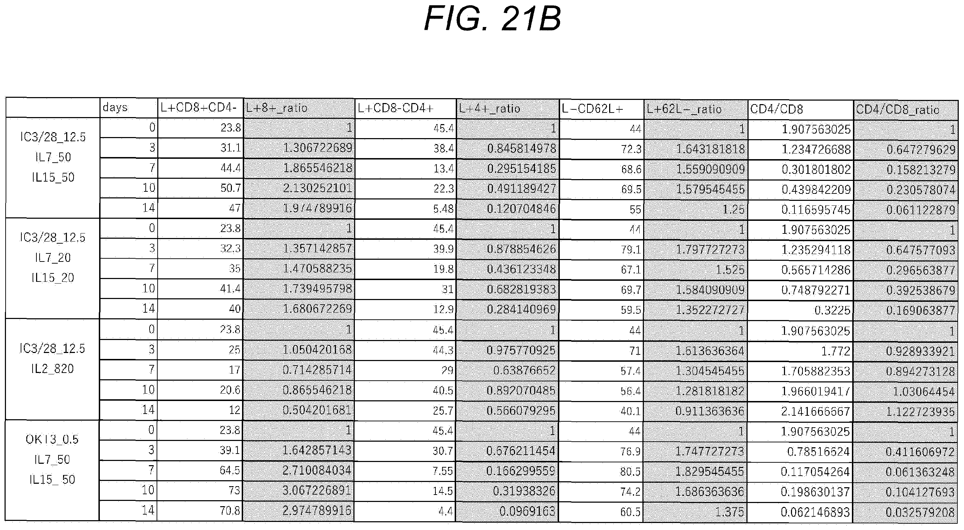

Using various PBMCs, the proliferation rate of each cell type in cultures cultured under various culture conditions was measured. A data set including the culture conditions used in the culture and the measured growth rate was created. An example of the data set is illustrated in Figs. 21A and B.

Fig. 21A illustrates data related to culture conditions. The data illustrated in the drawing includes cytokine stimulation data and antibody stimulation data.

In the drawing, for example, five rows belonging to "IC 3/28_12.5 IL7_50 IL15_50" correspond to one of the wells described above. Each of the lower five rows also corresponds to one well.

In the drawing, the "days" column represents the number of culture days, and 3, 7, 10, and 14 represent the days on which expansion culture and measurement with a flow cytometer were performed. The "OKT3" column and the "IC 3/28" column illustrate the antibody concentration in the medium, and the "IL2" column, the "IL7" column, and the "IL15" column illustrate the cytokine concentration in the medium. The "OKT3_accum." column and the "IC 3/28_accum." column illustrate the antibody concentration accumulated value in the medium, and the "IL2_accum." column, the "IL7_accum." column, and the "IL15_accum." column illustrate the cytokine concentration accumulated value in the medium.

The antibody concentration accumulated value is a value obtained by multiplying the "antibody concentration in the medium" by the "number of days cultured in the medium having the antibody concentration". The cytokine concentration accumulated value is a value obtained by multiplying the "cytokine concentration in the medium" by the "number of days cultured in the medium having the cytokine concentration".

As described above, the data set used in the present technology may include antibody stimulation data. The antibody stimulation data is particularly data based on the antibody concentration, and is preferably a cumulative value of the antibody concentration and the number of culture days in culture (that is, "antibody concentration" × "number of culture days").

In addition, the data set used in the present technology may include cytokine stimulation data. The cytokine stimulation data is particularly data based on the cytokine concentration, and is preferably a cumulative value of the cytokine concentration and the number of days of culture in culture (that is, "cytokine concentration" × "number of days of culture").

In addition, by using the cumulative value in this manner, a learned model in consideration of time can be generated, and the future state of the culture can be predicted based on the state of the culture at a certain time point. For example, since the above data includes a cumulative value calculated by the concentration × the number of days, the data can be used to generate a learned model for predicting how many days culture is required in a case where stimulation at a constant concentration is continued from the cumulative value. In addition, instead of the number of culture days, for example, another time unit such as hour or minute may be used.

Such culture condition data may be handled as treatment conditions in the present technology. In addition, such culture condition data may be used as an explanatory variable or an objective variable (in particular, an explanatory variable) for generating a learned model. For example, the culture condition data may be included in a data set for causing the first learner and/or the second learner according to the present technology to perform machine learning, and in particular, may be handled as an explanatory variable or an objective variable in the data set.

Fig. 21B illustrates data related to cultured cells. The data illustrated in the drawing includes data related to cell configuration.

In the drawing, for example, five rows belonging to "IC 3/28_12.5 IL7_50 IL15_50" correspond to one of the wells described above. Each of the lower five rows also corresponds to one well. Furthermore, the five rows belonging to "IC 3/28_12.5 IL7_50 IL15_50" correspond to the five rows belonging to "IC 3/28_12.5 IL7_50 IL15_50" described above with reference to Fig. 21A.

In the drawing, the "days" column represents the number of culture days, and 3, 7, 10, and 14 represent the days on which expansion culture and measurement with a flow cytometer were performed. In the drawing, the "L+ CD8+ CD4-" column, the "L+ CD8-CD4+" column, and the "L+ CD62L+" column represent the proportions of cell types in the cultured cell group, and respectively represent the proportion of CD8 positive cells (CD4 negative), the proportion of CD4 positive cells (CD8 negative), and the proportion of CD62L positive cells. Furthermore, the "CD4/CD8" column illustrates the ratio of the proportion of CD4 positive cells to the proportion of CD8 positive cells.

In the drawing, the "L+ 8+_ratio" column, the "L+ 4+_ratio" column, and the "L+ 62L+_ratio" column illustrate the change rate of the proportion of CD8 positive cells, the change rate of the proportion of CD4 positive cells, and the change rate of the proportion of CD62L positive cells, respectively. The change rate is a ratio of a ratio after the elapse of a predetermined number of days to the ratio at culture day 0 (that is, "composition ratio after elapse of a predetermined number of days"/"composition ratio at day 0").

Furthermore, the "CD4/CD8_ratio" column illustrates the change rate in the ratio of the proportion of CD4 positive cells to the proportion of CD8 positive cells. The change rate is a ratio (that is, "the CD4/CD8 ratio after a lapse of a predetermined number of days"/"the CD4/CD8 ratio on day 0") of the CD4/CD8 ratio after a lapse of a predetermined number of days to the CD4/CD8 ratio on culture day 0.

Such data related to cells may be handled as information regarding cells in the present technology, and in particular, may be handled as information regarding cells before or after treatment. The data related to the cell may be used as an explanatory variable or an objective function (particularly, an objective variable) for generating the learned model. For example, data related to the cultured cells may be included in a data set for causing the first learner and/or the second learner according to the present technology to perform machine learning, and in particular, may be handled as an explanatory variable or an objective variable in the data set.

### (1-3) Creation of Learned Model

Using the data set, a learned model of the cell configuration was created. Open source software that performs deep learning was used to create a learned model.

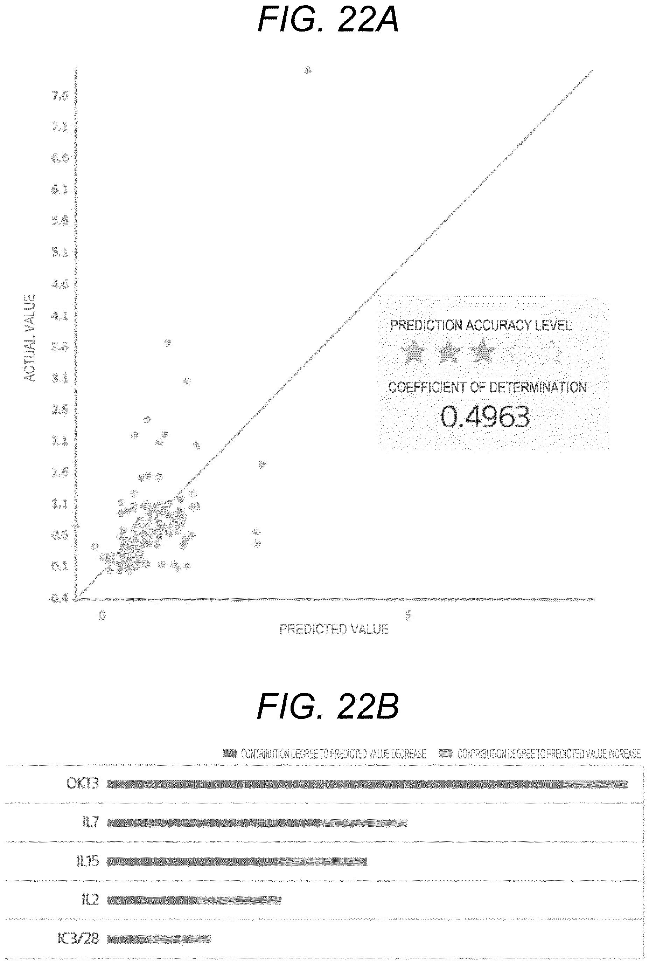

The machine learning software was caused to read a data set in which the explanatory variable was the cumulative value of the concentrations of IC 3/28, OKT3, IL2, IL7, and IL15 and the objective variable was the change rate of the composition ratio of CD4+ cells or the change rate of the composition ratio of CD8+ cells, thereby generating a learned model. The coefficient of determination of the generated learned model was 0.4963 (Fig. 22A). From this result, it can be seen that the composition ratio of the cell type after culture can be predicted based on the data related to the culture conditions. In addition, it can be seen that the prediction accuracy of the generated learned model is also relatively high.

Furthermore, with the generation of the learned model, the software generates contribution degree data indicating the contribution degree of each explanatory variable to the prediction accuracy (that is, the degree of contribution to the prediction accuracy). As the contribution degree data, bar graph data indicating the contribution degree of each explanatory variable with a bar is generated as illustrated in Fig. 22B. From the contribution degree data, for example, it can be seen that the contribution degree of OKT3 is large.

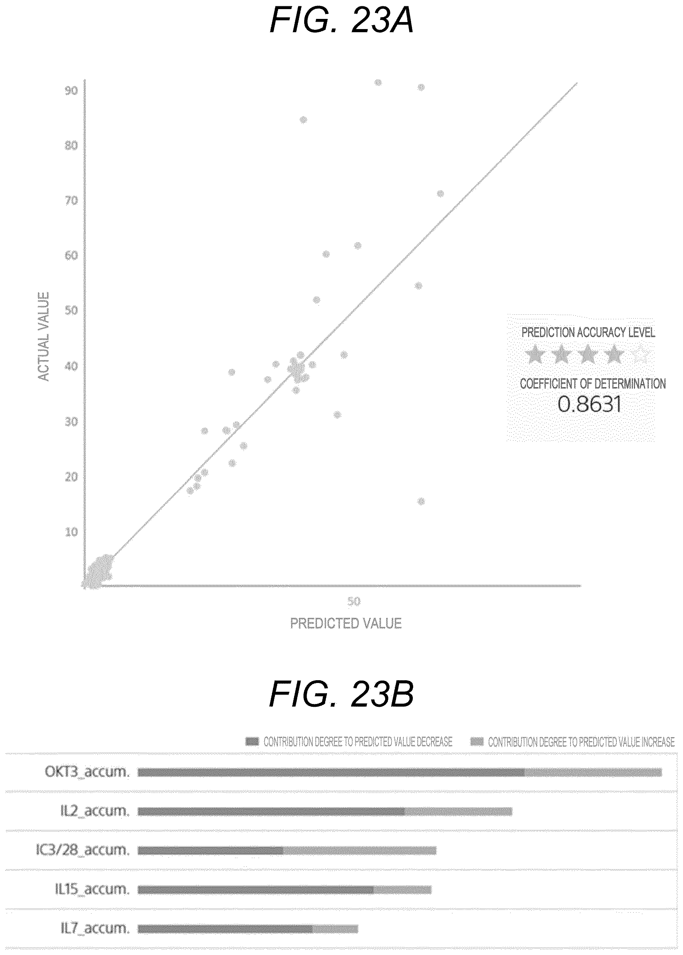

Next, the machine learning software was caused to read a data set in which the explanatory variable was the cumulative value of the concentrations of IC 3/28, OKT3, IL2, IL7, and IL15 and the change rate of the composition ratio of CD4+ cells, and the objective variable was the change rate of the composition ratio of CD8+ cells, thereby generating a learned model. The coefficient of determination of the generated learned model was 0.8631 (Fig. 23A). From this result, it can be seen that the prediction accuracy can be further improved by adopting the measured value related to the cultured cell as an explanatory variable. As described above, it was indicated that the ratio of specific cells after culture can be predicted using culture conditions and/or measured values.

Note that contribution degree data was also generated for the case illustrated in Fig. 23A. As the contribution degree data, bar graph data indicating the contribution degree of each explanatory variable with a bar is generated as illustrated in Fig. 23B.

Furthermore, it is conceivable that a learned model with higher prediction accuracy can be generated by predicting a culture result using a learned model generated using a certain data set, then acquiring an actual culture result, and then generating a learned model again using a data set obtained by adding data related to the acquired culture result (for example, culture conditions and cell composition of a culture obtained under the culture conditions) to the certain data set. That is, it can be seen that the learned model with higher prediction accuracy can be generated by updating the database in the cell treatment system according to the present technology.

The cell treatment system (particularly the information processing unit 2) according to the present technology may be configured to generate prediction accuracy data of a learned model generated as described above.

The prediction accuracy data may include, for example, data indicating the prediction accuracy of the learned model, such as the coefficient of determination described above. The prediction accuracy data can be output (for example, displayed) by, for example, an output device (for example, a display device) connected to the information processing unit. Thus, the user can grasp the prediction accuracy of the generated learned model.

As described above, the prediction accuracy data may include the contribution degree data of each explanatory variable. With the contribution degree data, the user can grasp treatment conditions important for the final culture result among the treatment conditions (for example, stimulating factors) adopted as explanatory variables.

Note that, the present disclosure can also have the following configuration.
[1] A cell treatment system including:
   a cell culture unit capable of culturing a cell group;
   a measurement unit capable of measuring the cell group; and
   an estimation unit, in which
   the estimation unit estimates, on the basis of information regarding a cell before treatment of the cell group and at least one of information regarding a predetermined cell to reach after treatment of the cell group or a predetermined treatment condition for the cell group before treatment, a treatment condition under which the cell group is derivable to the information regarding the predetermined cell or information regarding the cell after treatment of the cell group derived by the predetermined treatment condition.
[2] The cell treatment system according to [1], in which the estimation unit sets the information regarding the predetermined cell and/or the predetermined treatment condition on the basis of information regarding approval of the cell that is commercialized.
[3] The cell treatment system according to [1] or [2], in which the measurement unit is configured to acquire at least one of the information regarding the cell before treatment, the information regarding the cell after treatment, or information regarding the cell while the cell group is in treatment.
[4] The cell treatment system according to [3], in which the measurement unit includes at least one of an image acquisition unit or a signal detection unit.
[5] The cell treatment system according to [4], in which the image acquired by the image acquisition unit is a stained image and/or a non-stained image.
[6] The cell treatment system according to [5], in which the stained image includes a fluorescence image.
[7] The cell treatment system according to [5] or [6], in which the non-stained image includes at least one of a bright field image, a phase difference image, a polarized image, or an image that is identified from information learned from the non-stained image or the fluorescence image and is pseudo-stained for each cell feature.
[8] The cell treatment system according to any one of [4] to [7], in which the image acquisition unit acquires an image from at least one of a CMOS, a signal processing sensor, or an event detection sensor.
[9] The cell treatment system according to [1], further including a control unit that performs control to sort the cells on the basis of the predetermined treatment condition or the treatment condition estimated by the estimation unit.
[10] The cell treatment system according to [1], further including a control unit that performs control to culture the cell on the basis of the predetermined treatment condition or the treatment condition estimated by the estimation unit.
[11] The cell treatment system according to [1], further including a control unit that performs control to sort and culture the cell on the basis of the predetermined treatment condition or the treatment condition estimated by the estimation unit.
[12] The cell treatment system according to [9], in which the control unit sets a threshold value for the estimated treatment condition or the information regarding the treated cell, and performs processing of stopping the production of the cell and/or re-collecting the cell depending on the threshold value or depending on information specified by a user.
[13] The cell treatment system according to any one of [9] to [12], in which the control unit treats the cell fixed to the culture vessel via a light-selective linker in such a manner that the cells are sorted by optical control.
[14] The cell treatment system according to [10] or [11], in which the culture control controls at least one of a physicochemical environment or a physiological environment.
[15] The cell treatment system according to [14], in which the control of the physicochemical environment includes control by at least one of humidity, pH, osmotic pressure, oxygen partial pressure, or carbon dioxide partial pressure.
[16] The cell treatment system according to [14] or [15], in which the control of the physiological environment includes control by at least one of culture solution, a stimulating factor, a transcription factor, or cell density.
[17] The cell treatment system according to any one of [1] to [16], further including a first learner that learns relevance between the cell treated under the treatment condition estimated by the estimation unit and information regarding therapy using the cell as input information.
[18] The cell treatment system according to [17], in which the estimation unit estimates information regarding therapy of the cell after treatment of the cell group derived by the treatment condition on the basis of the first learner and the information regarding the cell before treatment.
[19] The cell treatment system according to any one of [1] to [18], further including a second learner that learns relevance between the information regarding the cell before treatment and the treatment condition by using, as input information, the information regarding the cell before treatment, the treatment condition estimated by the estimation unit, and the cell before treatment treated under the treatment condition.
[20] The cell treatment system according to [19], in which the estimation unit estimates a treatment condition under which the cell group is derivable to the information regarding the predetermined cell on the basis of the second learner and the information regarding the cell before treatment.
[21] The cell treatment system according to any one of [1] to [20], in which the information regarding the cells before treatment includes at least one of a configuration, a state, a number, a proportion, a type, an increase rate, a survival rate, genetic information, or information regarding a molecule of the cell before treatment.
[22] The cell treatment system according to [21], in which the information regarding the molecule includes at least one of a gene expression control factor such as a transcription factor or a transcription control factor, information regarding a molecular marker, or information regarding a cell surface antigen.
[23] The cell treatment system according to any one of [1] to [22], in which the information regarding the cell after treatment includes at least one of a configuration, a state, a number, a proportion, an increase rate, a survival rate, a response rate, a recurrence rate, or a side effect of the cell after treatment.
[24] The cell treatment system according to any one of [1] to [23], in which the treatment condition includes a culture condition related to at least one of a stimulating factor or a number of culture days.
[25] The cell treatment system according to any one of [1] to [24], in which the cell group includes a peripheral blood mononuclear cell fraction collected from a patient or a donor.
[26] An information processing device including:
   an estimation unit that estimates, on the basis of
   information regarding a cell before treatment of a cell group, and at least one of information regarding a predetermined cell to reach after treatment of the cell group or a predetermined treatment condition for the cell group before treatment,
   a treatment condition under which the cell group is derivable to the information regarding the predetermined cell, or information regarding the cells after treatment of the cell group derived by the predetermined treatment condition.
[27] A learning data creation method including learning, by using a cell treated under a treatment condition estimated by an information processing device and information regarding a therapy using the cell as input information, relevance between the cell and the information regarding the therapy.
[28] A learning data creation method including learning, by using information regarding a cell before treatment, a treatment condition estimated by an information processing device, and the cell before treatment treated under the treatment condition as input information, relevance between the information regarding the cell before treatment and the treatment condition.
[29] A cell treatment method, including:
   an estimation step of estimating, on the basis of information regarding a cell before treatment of a cell group included in a sample and at least one of information regarding a predetermined cell to reach after treatment of the cell group set in advance or a predetermined treatment condition for the cell group before treatment,
   a treatment condition under which the cell group is derivable to information regarding the predetermined cell or information regarding the cell after treatment of the cell group derived under the predetermined treatment condition.

## Claims

1. A cell treatment system, comprising:
a cell culture unit capable of culturing a cell group;
a measurement unit capable of measuring the cell group; and
an estimation unit, wherein
the estimation unit estimates, on a basis of information regarding a cell before treatment of the cell group and at least one of information regarding a predetermined cell to reach after treatment of the cell group or a predetermined treatment condition for the cell group before treatment, a treatment condition under which the cell group is derivable to the information regarding the predetermined cell or information regarding the cell after treatment of the cell group derived by the predetermined treatment condition.

2. The cell treatment system according to claim 1, wherein the estimation unit sets the information regarding the predetermined cell and/or the predetermined treatment condition on a basis of information regarding approval of the cell that is commercialized.

3. The cell treatment system according to claim 1, wherein the measurement unit is configured to acquire at least one of the information regarding the cell before treatment, the information regarding the cell after treatment, or information regarding the cell while the cell group is in treatment.

4. The cell treatment system according to claim 3, wherein the measurement unit includes at least one of an image acquisition unit or a signal detection unit.

5. The cell treatment system according to claim 4, wherein the image acquired by the image acquisition unit is a stained image and/or a non-stained image.

6. The cell treatment system according to claim 5, wherein the stained image includes a fluorescence image.

7. The cell treatment system according to claim 5, wherein the non-stained image includes at least one of a bright field image, a phase difference image, a polarized image, or an image that is identified from information learned from the non-stained image or the fluorescence image and is pseudo-stained for each cell feature.

8. The cell treatment system according to claim 4, wherein the image acquisition unit acquires an image from at least one of a CMOS, a signal processing sensor, or an event detection sensor.

9. The cell treatment system according to claim 1, further comprising a control unit that performs control to sort the cells on a basis of the predetermined treatment condition or the treatment condition estimated by the estimation unit.

10. The cell treatment system according to claim 1, further comprising a control unit that performs control to culture the cell on a basis of the predetermined treatment condition or the treatment condition estimated by the estimation unit.

11. The cell treatment system according to claim 1, further comprising a control unit that performs control to sort and culture the cell on a basis of the predetermined treatment condition or the treatment condition estimated by the estimation unit.

12. The cell treatment system according to claim 9, wherein the control unit sets a threshold value for the estimated treatment condition or the information regarding the treated cell, and performs processing of stopping the production of the cell and/or re-collecting the cell depending on the threshold value or depending on information specified by a user.

13. The cell treatment system according to claim 9, wherein the control unit treats the cell fixed to the culture vessel via a light-selective linker in such a manner that the cells are sorted by optical control.

14. The cell treatment system according to claim 10, wherein the culture control controls at least one of a physicochemical environment or a physiological environment.

15. The cell treatment system according to claim 14, wherein the control of the physicochemical environment comprises control by at least one of humidity, pH, osmotic pressure, oxygen partial pressure, or carbon dioxide partial pressure.

16. The cell treatment system according to claim 14, wherein the control of the physiological environment includes control by at least one of culture solution, a stimulating factor, a transcription factor, or cell density.

17. The cell treatment system according to claim 1, further comprising a first learner that learns relevance between the cell treated under the treatment condition estimated by the estimation unit and information regarding therapy using the cell as input information.

18. The cell treatment system according to claim 17, wherein the estimation unit estimates information regarding therapy of the cell after treatment of the cell group derived by the treatment condition on a basis of the first learner and the information regarding the cell before treatment.

19. The cell treatment system according to claim 1, further comprising a second learner that learns relevance between the information regarding the cell before treatment and the treatment condition by using, as input information, the information regarding the cell before treatment, the treatment condition estimated by the estimation unit, and the cell before treatment treated under the treatment condition.

20. The cell treatment system according to claim 19, wherein the estimation unit estimates a treatment condition under which the cell group is derivable to the information regarding the predetermined cell on a basis of the second learner and the information regarding the cell before treatment.

21. The cell treatment system according to claim 1, wherein the information regarding the cells before treatment includes at least one of a configuration, a state, a number, a proportion, a type, an increase rate, a survival rate, genetic information, or information regarding a molecule of the cell before treatment.

22. The cell treatment system according to claim 21, wherein the information regarding the molecule includes at least one of a gene expression control factor such as a transcription factor or a transcription control factor, information regarding a molecular marker, or information regarding a cell surface antigen.

23. The cell treatment system according to claim 1, wherein the information regarding the cell after treatment includes at least one of a configuration, a state, a number, a proportion, an increase rate, a survival rate, a response rate, a recurrence rate, or a side effect of the cell after treatment.

24. The cell treatment system according to claim 1, wherein the treatment condition includes a culture condition related to at least one of a stimulating factor or a number of culture days.

25. The cell treatment system according to claim 1, wherein the cell group includes a peripheral blood mononuclear cell fraction collected from a patient or a donor.

26. An information processing device comprising:
an estimation unit that estimates, on a basis of
information regarding a cell before treatment of a cell group, and at least one of information regarding a predetermined cell to reach after treatment of the cell group or a predetermined treatment condition for the cell group before treatment,
a treatment condition under which the cell group is derivable to the information regarding the predetermined cell, or information regarding the cells after treatment of the cell group derived by the predetermined treatment condition.

27. A learning data creation method comprising learning, by using a cell treated under a treatment condition estimated by an information processing device and information regarding a therapy using the cell as input information, relevance between the cell and the information regarding the therapy.

28. A learning data creation method comprising learning, by using information regarding a cell before treatment, a treatment condition estimated by an information processing device, and the cell before treatment treated under the treatment condition as input information, relevance between the information regarding the cell before treatment and the treatment condition.

29. A cell treatment method, comprising:
an estimation step of estimating, on a basis of information regarding a cell before treatment of a cell group included in a sample and at least one of information regarding a predetermined cell to reach after treatment of the cell group set in advance or a predetermined treatment condition for the cell group before treatment,
a treatment condition under which the cell group is derivable to information regarding the predetermined cell or information regarding the cell after treatment of the cell group derived under the predetermined treatment condition.
